# EUROPEAN PATENT APPLICATION

(11) **EP 2 444 088 A1**
(43) Date of publication of application: **25.04.2012**
(21) Application number: 10382277.1
(22) Date of filing: 22.10.2010
(51) Int. Cl.: A61K 31/455, A61K 31/505, A61P 17/00, A61P 35/00

(54) **Amino derivatives for the treatment of proliferative skin disorders**

(71) Applicant: Almirall, S.A., 08022 Barcelona (ES)
(72) Inventor: Godessart Marina, Nuria, 08980, SANT FELIU DE LLOBREGAT (ES); Balague Pelaez, Cristina, 08980, Sant Feliu de Llobregat (ES)
(74) Representative: Elzaburu Marquez, Alberto

(57) **Abstract**

Compositions comprising specific amino derivatives for their use in treating proliferative skin diseases or disorders and the use of specific amino derivatives in such a treatment is disclosed.

## Description

### FIELD OF THE INVENTION

The present invention relates to methods and compositions for the treatment of proliferative skin diseases or disorders, and more particularly, to compositions comprising specific amino derivatives for their use in treating proliferative skin diseases or disorders and to the use of specific amino derivatives in such a treatment.

### BACKGROUND OF THE INVENTION

The skin is the largest organ of the body, covering the entire outside of the body, and comprises the epidermis, dermis, and subcutaneous layers. Numerous disorders of the skin are known, ranging from those which merely cause discomfort or psychological stress, such as rashes, to those which are life threatening, such as skin cancer.

Skin cancer is the most common form of cancer in some countries such as Australia or the United States. Skin cancers are classified by the types of epidermal cells involved. Basal cell carcinoma develops from abnormal growth of the cells in the lowest layer of the epidermis and is the most common type of skin cancer; squamous cell carcinoma (SCC) involves changes in the squamous cells, found in the middle layer of the epidermis; and melanoma occurs in the melanocytes. Skin melanoma is less common than squamous or basal cell carcinoma (BCC), but more dangerous. It is the leading cause of death from skin disease.

Actinic (syn. solar) keratosis (AK) is a premalignant skin disorder that represents the initial manifestation of an abnormal keratinocyte proliferation. AK lesions exhibits many of the cellular changes observed in squamous cell carcinoma. Although most actinic keratosis lesions do not actually become cancerous, some lesions can become malignant.

Current treatments for actinic keratosis consist of operative and physical methods and topical pharmacological therapies and include (Guidelines for the management of AKs, D. de Berker et al., British Journal of Dermatology, 2007, 156, pp 222-230):
- Cryosurgery, cryosurgery using liquid nitrogen is the most common modality for treating actinic keratoses, although compressed nitrous oxide or carbon dioxide is also used. Liquid nitrogen is sprayed directly on the lesions or applied using a cotton-tipped swab.
- Curettage or excisional surgery, which involves mechanically scraping away abnormal tissue using a sharp curette.
- Photodynamic therapy, which involves applying a photosensitizing agent to each actinic keratosis, followed by exposure to light of a specific wavelength; this leads to cell death.
- Topical therapies, several topical therapies are available for the treatment of actinic keratoses, including various salicylic acid ointment, 5-fluorouracil formulations, imiquimod 5% cream (Aldara), diclofenac 3% gel (Solaraze), tretinoin cream or masoprocol cream.

On the other hand, the enzyme dihydroorotate dehydrogenase (DHODH) is the enzyme that catalyzes the fourth step in the pyrimidine biosynthetic pathway namely the conversion of dihydroorotate to orotate concomitantly with a electron transfer to ubiquinone (cofactor Q) via a flavin mononucleotide intermediate (Loffler et al Mol Cell Biochem, 1997). In contrast to parasites (Plasmodium falciparum) (McRobert et al Mol Biochem Parasitol 2002) and bacteria (E.coli) which exclusively have this de novo pathway as the source of pyrimidines, mammal cells have an additional salvage pathway.

During homeostatic proliferation, the salvage pathway which is independent of DHODH seems sufficient for the cellular supply with pyrimidine bases. Only, cells with a high turnover and particularly T and B lymphocytes need the de novo pathway to proliferate. In these cells, DHODH inhibition stops the cell cycle progression suppressing DNA synthesis and consequently cell proliferation (Breedveld FC et al, Ann Rheum Dis 2000).

The DHODH enzyme has recently been reported to be consistently expressed in premalignant keratinocytes (HaCat cells) as well as in cutaneous squamous cell carcinoma cell lines (COLO16 cell line derived from a metastatic squamous cell carcinoma, and the SCC-13, SRB-1, and SRB-2 cell lines derived from skin carcinoma in situ). The levels of DHODH expression among those cutanous keratinocytes was similar to that observed in premalignant (PWR-1 E) as well as malignant prostate epithelial cells (DU-145, LNCaP and PC-3). These results suggest that DHODH could be an essential constituent in skin and prostate tumorigenesis. Teriflunomide (2-Butenamide, 2-cyano-3-hydroxy-N-[4-(trifluoromethyl)phenyl]-; CAS RN 108605-62-5), which is the active metabolite of Leflunomide (4-Isoxazolecarboxamide, 5-methyl-N-[4-(trifluoromethyl)phenyl]-; CAS RN 75706-12-6) sold under the trade name Arava for the treatment of rheumatoid arthritis, has been reported to induce a dose- and time-dependent cytostasis on premalignant and malignant human cutaneous keratinocytes, which was followed by apoptosis (Hial N et. al., Apoptosis 2010).

Considering that skin diseases or disorders represent a spectrum of clinical complaint and pathology and treatment should be considered on an individual basis according to signs, symptoms and history, there is a need for alternative treatments of said diseases.

Surprisingly, it has been found that certain amino derivatives, which are DHODH inhibitors, are more potent than teriflunomide in preventing the abnormal proliferation of human cutaneous keratinocytes and, therefore, are useful in the treatment of proliferative skin diseases or disorders.

### SUMMARY OF THE INVENTION

The present invention therefore provides specific amino derivatives of formula (I) A-NH-B, or a pharmaceutically acceptable salt or N-oxide or solvate or deuterated derivative thereof, for use in treating proliferative skin diseases or disorders, wherein
A represents a group of formula A1 or A2, 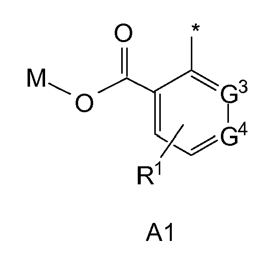 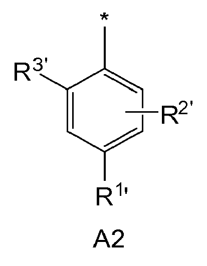 wherein the asterisk symbol indicates the bond that is linked to the -NH-B moiety; and wherein:
   - in the group of formula A1:
      - R¹ represents a group selected from hydrogen atoms, halogen atoms, C₁₋₄ alkyl groups which may be optionally substituted by 1, 2 or 3 substituents selected from halogen atoms and hydroxy groups, and C₃₋₈ cycloalkyl groups which may be optionally substituted by 1, 2 or 3 substituents selected from halogen atoms and hydroxy groups;
      - one of the groups G³ and G⁴ is a nitrogen atom and the other is a CH group;
      - M is a hydrogen atom or a pharmaceutically acceptable cation;
   - in the group of formula A2:
      - R¹' is selected from the group consisting of hydrogen atoms, halogen atoms, C₁₋₄ alkyl, C₃₋₄ cycloalkyl, -CF₃ and -OCF₃;
      - R²' is selected from the group consisting of hydrogen atoms, halogen atoms and C₁₋₄ alkyl group;
      - R³' is selected from the group consisting of -COOR⁵' -CONHR⁵', tetrazolyl, -SO₂NHR⁵' and -CONHSO₂R⁵' groups, wherein R⁵' is selected from the group consisting of a hydrogen atom and linear or branched C₁₋₄ alkyl groups;
B represents a group of formula B1 or B2, 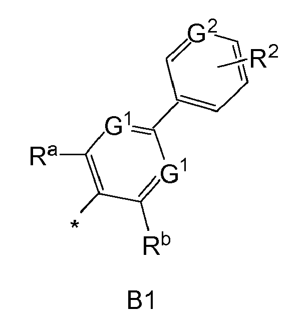 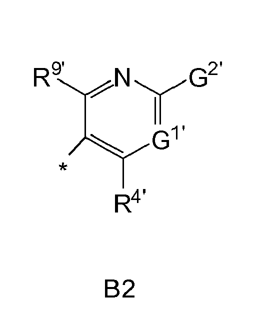 wherein the asterisk symbol indicates the bond that is linked to the A-NH-moiety; and wherein:
   - in the group of formula B1
      - one of the groups G¹ represents a nitrogen atom or a group CR^{c} and the other represents a group CR^{c};
      - G² represents a nitrogen atom or a group CR^{d};
      - R² represents a group selected from hydrogen atoms, halogen atoms, hydroxyl groups, C₁₋₄ alkyl groups which may be optionally substituted by 1, 2 or 3 substituents selected from halogen atoms and hydroxy groups, C₁₋₄ alkoxy groups which may be optionally substituted by 1, 2 or 3 substituents selected from halogen atoms and hydroxy groups, and C₃₋₈ cycloalkyl groups which may be optionally substituted by 1, 2 or 3 substituents selected from halogen atoms and hydroxy groups;
      - R^{a}, R^{b} and R^{c} independently represent groups selected from hydrogen atoms, halogen atoms, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups;
      - R^{d} represents a group selected from hydrogen atoms, halogen atoms, hydroxyl groups, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups, and C₃₋₈ cycloalkoxy groups which may be optionally substituted by 1, 2 or 3 substituents selected from halogen atoms and hydroxy groups;
      with the proviso that, when at least one of the groups R^{a} and R^{b} represent a hydrogen atom and G² is a group CR^{d}, then R^{d} represents a group selected from C₁₋₄ alkoxy groups which may be optionally substituted by 1, 2 or 3 substituents selected from halogen atoms and hydroxy groups, C₃₋₈ cycloalkoxy groups which may be optionally substituted by 1, 2 or 3 substituents selected from halogen atoms and hydroxy groups;
   - in the group of formula B2
      - R⁴' is selected from the group consisting of a hydrogen atom, and a C₁₋₄ alkyl group;
      - R⁹' is selected from the group consisting of a hydrogen atom and a phenyl group;
      - G¹' represents a group selected from N and CR⁶' wherein R⁶' is selected from the group consisting of hydrogen atoms, halogen atoms, C₁₋₄ alkyl, C₃₋₄ cycloalkyl, C₁₋₄ alkoxy, -CF₃, -OCF₃, monocyclic N-containing C₅₋₇ heteroaryl, monocyclic N-containing C₃₋₇ heterocyclyl groups and a C₆₋₁₀ aryl group which is optionally substituted with one or more substituents selected from halogen atoms and a C₁₋₄ alkyl group;
      - G²' represents a group selected from:
      - a hydrogen atom, a hydroxy group, a halogen atom, a C₃₋₄ cycloalkyl group, a C₁₋₄ alkoxy group and -NR^{a}'R^{b}', wherein R^{a}' represents a C₁₋₄ alkyl group and R^{b}' is selected from a group consisting of C₁₋₄ alkyl group and C₁₋₄ *alkoxy-C₁₋₄ alkyl group, or R^{a}' and R^{b}' together with the nitrogen atom to which they are attached form a saturated 6 to 8 membered heterocyclic ring optionally containing one oxygen atom as an additional heteroatom;
      - a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷'R⁸', wherein R⁷' and R⁸' are independently selected from hydrogen atom, linear or branched C₁₋₄ alkyl group, C₃₋₇ cycloalkyl group, or R⁷' and R⁸' together with the nitrogen atom to which they are attached form a group of formula 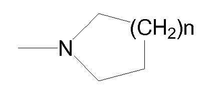 wherein n is an integer from 0 to 3;
         and
      - a phenyl group which is optionnally substituted by one or more substituents selected from halogen atoms, C₁₋₄ alkyl, hydroxy, C₁₋₄ alkoxy, C₃₋₄ cycloalkyl, C₃₋₄ cycloalkoxy, cyano, -CF₃, -OCF₃, -CONR₇'R₈', oxadiazolyl, triazolyl, pyrazolyl and imidazolyl groups, wherein the oxadiazolyl, triazolyl, pyrazolyl and imidazolyl groups are optionally substituted by C₁₋₄ alkyl or C₃₋₇ cycloalkyl group and wherein R₇' and R₈' are independently selected from hydrogen atom, linear or branched C1-4 alkyl group, a C₃₋₇ cycloalkyl group, or R₇' and R₈' together with the nitrogen atom to which they are attached form a group of formula 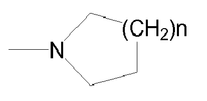 wherein n is an integer from 0 to 3;
      - or, when G¹' represents CR⁶', G²' together with R⁶' form a non-aromatic C₅₋₁₀ carbocyclic group or a C₆₋₁₀ aryl group.

Typically, the disease or disorder is selected from a) epidermal and follicular tumors (such as basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma); b) sebaceous gland tumors (such as sebaceous carcinoma); c) sweat gland tumors (such as mammary Paget's disease, extramammary Paget's disease, eccrine porocarcinoma, microcystic adnexal carcinoma (MAC) or mucinous carcinoma of the skin); d) nervous system tumors (such as Merkel cell carcinoma (MCC)); e) mesenchymal tumors (such as dermatofibrosarcoma protuberans (DFSP), malignant fibrous histiocytoma (MFH), atypical fibroxanthoma, epithelioid sarcoma, synovial sarcoma, liposarcoma, angiosarcoma, Kaposi's sarcoma, cutaneous lymphoma or multiple myeloma); f) metastatic carcinoma of the skin; g) skin melanoma; h) lamellar ichthyosis; i) acne; or j) hirsutism.

The invention further relates to a topical pharmaceutical composition comprising an amino derivative of formula (I) A-NH-B alone or in combination with one or more active ingredients for use in the treatment of proliferative skin diseases or disorders, in particular wherein the disease or disorder is as defined above.

The invention further relates to a method for treating a subject afflicted with a proliferative skin disease, which comprises applying to the affected area of the skin of said subject an effective amount of an amino derivative of formula (I) A-NH-B, or a pharmaceutical composition comprising an amino derivative of formula (I) A-NH-B alone or in combination with one or more active ingredients.

The invention further relates to the use of an amino derivative of formula (I) A-NH-B for the manufacture of a medicament for the treatment of a proliferative skin disease or disorder, in particular wherein the disease or disorder is as defined above.

### DETAILED DESCRIPTION OF THE INVENTION

The amino derivatives of formula (I) A-NH-B

Typically, when A represents a group of formula A1, B represents a group of formula B1. Typically, when A represents a group of formula A2, B represents a group of formula B2.

More typically, the amino derivatives of the invention, which are DHODH inhibitors, are selected from the group consisting of amino(iso)nicotinic acid derivatives of formula (I-a) 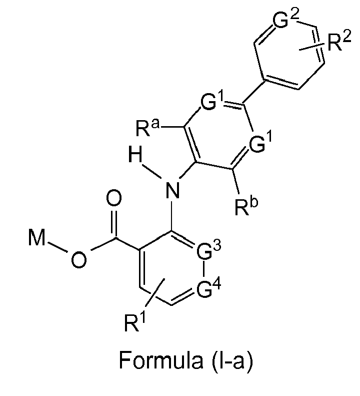 wherein
- one of the groups G¹ represents a nitrogen atom or a group CR^{c} and the other represents a group CR^{c};
- G² represents a nitrogen atom or a group CR^{d};
- R¹ represents a group selected from hydrogen atoms, halogen atoms, C₁₋₄ alkyl groups which may be optionally substituted by 1, 2 or 3 substituents selected from halogen atoms and hydroxy groups, and C₃₋₈ cycloalkyl groups which may be optionally substituted by 1, 2 or 3 substituents selected from halogen atoms and hydroxy groups;
- R² represents a group selected from hydrogen atoms, halogen atoms, hydroxyl groups, C₁₋₄ alkyl groups which may be optionally substituted by 1, 2 or 3 substituents selected from halogen atoms and hydroxy groups, C₁₋₄ alkoxy groups which may be optionally substituted by 1, 2 or 3 substituents selected from halogen atoms and hydroxy groups, and C₃₋₈ cycloalkyl groups which may be optionally substituted by 1, 2 or 3 substituents selected from halogen atoms and hydroxy groups;
- R^{a}, R^{b} and R^{c} independently represent groups selected from hydrogen atoms, halogen atoms, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups;
- R^{d} represents a group selected from hydrogen atoms, halogen atoms, hydroxyl groups, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups, and C₃₋₈ cycloalkoxy groups which may be optionally substituted by 1, 2 or 3 substituents selected from halogen atoms and hydroxy groups;
- one of the groups G³ and G⁴ is a nitrogen atom and the other is a CH group;
- M is a hydrogen atom or a pharmaceutically acceptable cation;
with the proviso that, when at least one of the groups R^{a} and R^{b} represent a hydrogen atom and G² is a group CR^{d}, then R^{d} represents a group selected from C₁₋₄ alkoxy groups which may be optionally substituted by 1, 2 or 3 substituents selected from halogen atoms and hydroxy groups, C₃₋₈ cycloalkoxy groups which may be optionally substituted by 1, 2 or 3 substituents selected from halogen atoms and hydroxy groups;
or a pharmaceutically acceptable salt, or solvate, or N-oxide, or stereoisomer or deuterated derivate thereof;
or azabiphenylaminobenzoic acid derivatives of formula (I-b) 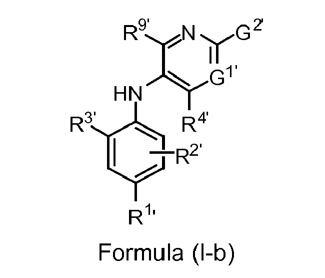 wherein:
- R¹' is selected from the group consisting of hydrogen atoms, halogen atoms, C₁₋₄ alkyl, C₃₋₄ cycloalkyl, -CF₃ and -OCF₃;
- R²' is selected from the group consisting of hydrogen atoms, halogen atoms and C₁₋₄ alkyl group;
- R³' is selected from the group consisting of -COOR⁵', -CONHR⁵', tetrazolyl, -SO₂NHR⁵' and -CONHSO₂R⁵' groups, wherein R⁵' is selected from the group consisting of a hydrogen atom and linear or branched C₁₋₄ alkyl groups;
- R⁴' is selected from the group consisting of a hydrogen atom, and a C₁₋₄ alkyl group;
- R⁹' is selected from the group consisting of a hydrogen atom and a phenyl group;
- G¹' represents a group selected from N and CR⁶' wherein R⁶' is selected from the group consisting of hydrogen atoms, halogen atoms, C₁₋₄ alkyl, C₃₋₄ cycloalkyl, C₁₋₄ alkoxy, -CF₃, -OCF₃, monocyclic N-containing C₅₋₇ heteroaryl, monocyclic N-containing C₃₋₇ heterocyclyl groups and a C₆₋₁₀ aryl group which is optionally substituted with one or more substituents selected from halogen atoms and a C₁₋₄ alkyl group;
- G²' represents a group selected from:
   - a hydrogen atom, a hydroxy group, a halogen atom, a C₃₋₄ cycloalkyl group, a C₁₋₄ alkoxy group and -NR^{a}'R^{b}', wherein R^{a}' represents a C₁₋₄ alkyl group and R^{b}' is selected from a group consisting of C₁₋₄ alkyl group and C₁₋₄ alkoxy-C₁₋₄ alkyl group, or R^{a}' and R^{b}' together with the nitrogen atom to which they are attached form a saturated 6 to 8 membered heterocyclic ring optionally containing one oxygen atom as an additional heteroatom;
   - a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷'R⁸', wherein R⁷' and R⁸' are independently selected from hydrogen atom, linear or branched C₁₋₄ alkyl group, C₃₋₇ cycloalkyl group, or R⁷' and R⁸' together with the nitrogen atom to which they are attached form a group of formula 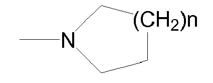 wherein n is an integer from 0 to 3;
      and
- a phenyl group which is optionnally substituted by one or more substituents selected from halogen atoms, C₁₋₄ alkyl, hydroxy, C₁₋₄ alkoxy, C₃₋₄ cycloalkyl, C₃₋₄ cycloalkoxy, cyano, -CF₃, -OCF₃, -CONR₇'R₈', oxadiazolyl, triazolyl, pyrazolyl and imidazolyl groups, wherein the oxadiazolyl, triazolyl, pyrazolyl and imidazolyl groups are optionally substituted by C₁₋₄ alkyl or C₃₋₇ cycloalkyl group and wherein R₇' and R₈' are independently selected from hydrogen atom, linear or branched C1-4 alkyl group, a C₃₋₇ cycloalkyl group, or R₇' and R₈' together with the nitrogen atom to which they are attached form a group of formula 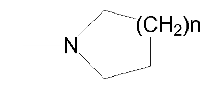 wherein n is an integer from 0 to 3;
- or, when G¹' represents CR⁶', G²' together with R⁶' form a non-aromatic C₅₋₁₀ carbocyclic group or a C₆₋₁₀ aryl group;
or a pharmaceutically acceptable salt, or solvate, or N-oxide, or stereoisomer or deuterated derivate thereof.

As used herein, a DHODH inhibitor is typically a compound which has an hDHODH IC₅₀ of about 10 µM or less, preferably 5 µM or less, more preferably 1 µM or less, as measured using a chromogen reduction assay with DCIP (2,6-dichlorophenol-indophenol).

Typically, in the chromogen reduction assay, the substrate oxidation (Dihydroorotate, L-DHO), as well as cosubstrate reduction (coenzyme Q, CoQ) is coupled to the chromogen reduction, and enzymatic activity results in a loss of chromogen absorbance at 600 nm.

Preferably, the chromogen reduction assay comprises:
- incubating enzyme extracts (typically 8 µL, ~1.5 µg of human protein), preferably in 96 well plates;
- preparing an assay mixture (typically 200 µL) containing 200 µM CoQD, 100 µM L-DHO, 120 µM DCIP in the assay buffer (which assay buffer typically contains 100 mM HEPES pH 8.0, 150 mM NaCl, 10% Glicerol, 0.05% Triton X-100) and 2 µL of test compound, wherein the compound is dissolved in an appropriate solvent, typically DMSO at a stock concentration of 1 mM,
- contacting the enzyme and the assay mixture;
- incubating the enzyme and the assay mixture, typically for 10 min at room temperature;
- measuring DCIP reduction by counting a decrease in absorbance at 600 nm, typically using Spectramax instrumentation; and
- repeating using different concentrations of test compound, typically varying from 10 µM to 1 pM, to calculate an IC₅₀ (concentration of inhibitor required for 50% of inhibition).

Typically, all reactions are carried out in duplicate and graphs, determining IC₅₀ values for a compound, are plotted using the ABase software.

Preferably, the amino derivatives of the invention, which are DHODH inhibitors, have an hDHODH IC₅₀ of about 500 nM or less, preferably 300 nM or less, more preferably 200 nM or less, as measured using a chromogen reduction assay with DCIP (2,6-dichlorophenol-indophenol).

### The amino(iso)nicotinic acid derivatives of formula (I-a)

In an embodiment, the amino derivative of formula (I) A-NH-B is an amino(iso)nicotinic acid derivative of formula (I-a): 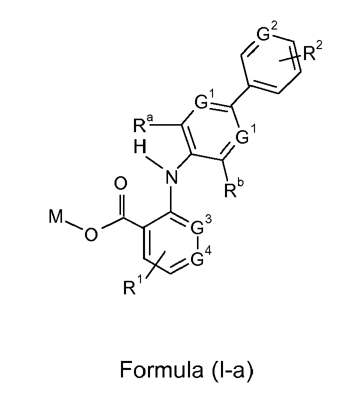 wherein
- one of the groups G¹ represents a nitrogen atom or a group CR^{c} and the other represents a group CR^{c};
- G² represents a nitrogen atom or a group CR^{d};
- R¹ represents a group selected from hydrogen atoms, halogen atoms, C₁₋₄ alkyl groups which may be optionally substituted by 1, 2 or 3 substituents selected from halogen atoms and hydroxy groups, and C₃₋₈ cycloalkyl groups which may be optionally substituted by 1, 2 or 3 substituents selected from halogen atoms and hydroxy groups;
- R² represents a group selected from hydrogen atoms, halogen atoms, hydroxyl groups, C₁₋₄ alkyl groups which may be optionally substituted by 1, 2 or 3 substituents selected from halogen atoms and hydroxy groups, C₁₋₄ alkoxy groups which may be optionally substituted by 1, 2 or 3 substituents selected from halogen atoms and hydroxy groups, and C₃₋₈ cycloalkyl groups which may be optionally substituted by 1, 2 or 3 substituents selected from halogen atoms and hydroxy groups;
- R^{a}, R^{b} and R^{c} independently represent groups selected from hydrogen atoms, halogen atoms, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups;
- R^{d} represents a group selected from hydrogen atoms, halogen atoms, hydroxyl groups, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups, and C₃₋₈ cycloalkoxy groups which may be optionally substituted by 1, 2 or 3 substituents selected from halogen atoms and hydroxy groups;
- one of the groups G³ and G⁴ is a nitrogen atom and the other is a CH group;
- M is a hydrogen atom or an pharmaceutically acceptable cation;
with the proviso that, when at least one of the groups R^{a} and R^{b} represent a hydrogen atom and G² is a group CR^{d}, then R^{d} represents a groups selected from C₁₋₄ alkoxy groups which may be optionally substituted by 1, 2 or 3 substituents selected from halogen atoms and hydroxy groups, C₃₋₈ cycloalkoxy groups which may be optionally substituted by 1, 2 or 3 substituents selected from halogen atoms and hydroxy groups;
and the pharmaceutically acceptable salt, or solvate, or N-oxide, or stereoisomer or deuterated derivate thereof.

The amino(iso)nicotinic acid derivatives of formula (I-a) and methods for their preparation are described in the International Patent Application No. W02008/077639.

As used herein the term alkyl embraces linear or branched hydrocarbon radicals having 1 to 4 carbon atoms. Preferred substituents on the alkyl groups are halogen atoms and hydroxy groups, and are more preferably halogen atoms.

Examples include methyl, ethyl, *n*-propyl, *i*-propyl, *n*-butyl, *sec*-butyl and *tert*-butyl radicals.

As used herein the term alkoxy embraces linear or branched oxy-containing radicals each having 1 to 4 carbon atoms. Preferred substituents on the alkoxy groups are halogen atoms and hydroxy groups, and are more preferably halogen atoms.

Examples include methoxy, ethoxy, *n*-propoxy, *i*-propoxy, *n*-butoxy, *sec*-butoxy and tert-butoxy radicals.

As used herein, the term cycloalkyl embraces saturated carbocyclic radicals and, unless otherwise specified, a cycloalkyl radical typically has from 3 to 8 carbon atoms.

Examples include cyclopropyl, cyclobutyl, cyclopentyl, cyclohexyl and cycloheptyl. When a cycloalkyl radical carries 2 or more substituents, the substituents may be the same or different. Preferred substiuents on the cycloalkyl groups are halogen atoms and hydroxy groups, and are more preferably halogen atoms.

As used herein, the term cycloalkoxy embraces saturated oxy-containing carbocyclic radicals and, unless otherwise specified, a cycloalkoxy radical typically has from 3 to 8 carbon atoms.

Examples include cyclopropyloxy, cyclobutyloxy, cyclopentyloxy, cyclohexyloxy and cycloheptyloxy. When a cycloalkoxy radical carries 2 or more substituents, the substituents may be the same or different. Preferred substituents on the cycloalkoxy groups are halogen atoms and hydroxy groups, and are more preferably halogen atoms.

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms typically a fluorine, chlorine or bromine atom, most preferably bromine or fluorine. The term halo when used as a prefix has the same meaning.

M may be a hydrogen atom or a pharmaceutically acceptable cation. When M is a pharmaceutically acceptable cation, the compound represented by formula (I-a) may alternatively be represented by formula (I-a*) below. 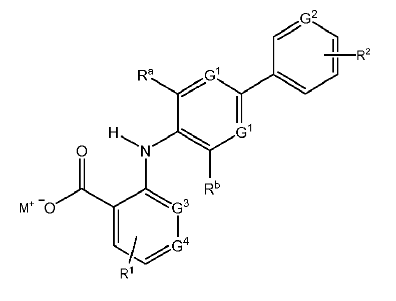 (I-a*)

As used herein, the term pharmaceutically acceptable cation embraces both inorganic cations, for example alkali metal cations (Li⁺, Na⁺, K⁺), alkaline earth cations (Ca²⁺, Mg²⁺) and other pharmaceutically acceptable inorganic cations known in the art (Zn²⁺, Al³⁺), and organic cations, for example ammonium ion (i.e., NH₄⁺) and substituted ammonium ions, such as NH₃R¹⁺, NH₂(R¹)₂⁺, NH(R¹)₃⁺ and N(R¹)₄⁺, where each R¹ is independently selected from a phenyl group, a benzyl group, C₁₋₄ alkyl and C₃₋₈ cycloalkyl.

Examples of some suitable substituted ammonium ions are EtNH₃⁺, Et₂NH₂⁺, Et₃NH⁺, (C₆H₁₁)₂NH₂⁺, CH₃CH₂CH₂CH₂NH₃⁺, PhCH₂NH₃⁺ and (Ph)(PhCH₂)NH₂⁺. An example of a common quaternary ammonium ion is N(CH₃)₄⁺.

Typically, M is a hydrogen atom or a pharmaceutically acceptable cation selected from Li⁺, Na⁺, K⁺, Ca²⁺ and Mg²⁺. It is preferred that M is a hydrogen atom or a pharmaceutically acceptable cation selected from Li⁺, Na⁺ and K⁺. More preferably M is a hydrogen atom or Li⁺, and most preferred is when M is a hydrogen atom.

If M of formula (I-a) is a pharmaceutically acceptable cation having a charge greater than +1, then additional anions are present to maintain the electroneutrality of the compound. The counteranion may be an anion X- as defined below or an anion as represented in formula (I-a*) above.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

As used herein, the term deuterated derivative embraces compounds of the invention where in a particular position at least one hydrogen atom is replaced by deuterium. Deuterium (D or 2H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

Typically, R¹ is selected from the group consisting of hydrogen, bromine and fluorine atoms, methyl, ethyl, cyclopropyl and cyclobutyl.

Typically, G³ represents a nitrogen atom and G⁴ represents a group CH.

Typically, G³ represents a group CH and G⁴ represents a nitrogen atom.

Typically, G¹ represent a group CR^{c}.

Typically, each R^{c} is independently selected from the groups consisting of hydrogen atoms, fluorine atoms, chlorine atoms and C₁₋₃ alkyl groups.

Typically, G² represents a group CR^{d}.

Typically, R^{d} is selected from the groups consisting of hydroxy, C₁₋₃ alkoxy groups, 2,2,2-trifluoroethoxy and C₃₋₄ cycloalkoxy groups. Preferably, C₁₋₃ alkoxy groups, 2,2,2-trifluoroethoxy and C₃₋₄ cycloalkoxy groups.

Typically, R^{a} is selected from the groups consisting of fluorine atoms, methyl groups and trifluoromethoxy groups.

Typically, R^{b} is selected from the group consisting of hydrogen atoms, fluorine atoms and chlorine atoms.

Typically, R² is selected from the group consisting of hydrogen atoms and halogen atoms, preferably hydrogen atoms and fluorine atoms.

Typically, both groups G¹ represent C(R^{c}) groups, G² represents a C(R^{d}) group, preferably G² is a group selected from C(OH), C(OMe) and C(OEt); R^{a} is selected from the group consisting of fluorine atoms and 2,2,2-trifluoroethoxy groups, R^{b} is selected from the group consisting of hydrogen atoms and fluorine atoms and R¹ is selected from the group consisting of hydrogen, bromine and fluorine atoms, methyl, ethyl and cyclopropyl groups; preferably, both G¹ represent CH groups, G² is a group selected from C(OMe) and C(OEt); R^{a} is a fluorine atom, R^{b} is selected from the group consisting of hydrogen atoms and fluorine atoms and R¹ is selected from the group consisting of hydrogen, bromine and fluorine atoms, methyl, ethyl and cyclopropyl groups.

Preferably, R^{c} is a hydrogen atom, R^{d} is a hydroxy or a C₁₋₃ alkoxy groups and R² is a hydrogen atom, preferably R^{c} is a hydrogen atom, R^{d} is a C₁₋₃ alkoxy and R² is a hydrogen atom.

Preferably, G³ represents a nitrogen atom, G⁴ represents a group CH and R^{b} is a fluorine atom and the compounds wherein G³ represents a group CH, G⁴ represents a nitrogen atom.

Preferred compounds of formula (I-a) are those in which both groups G¹ represent C(R^{c}) groups, G² represents a C(R^{d}) group, R^{a} is selected from the group consisting of hydrogen atoms, fluorine atoms, chlorine atoms, methyl groups, trifluoromethoxy groups and 2,2,2-trifluoroethoxy groups, R^{b} is selected from the group consisting of hydrogen atoms and fluorine atoms, R¹ is selected from the group consisting of hydrogen, bromine chlorine and fluorine atoms, methyl, ethyl and cyclopropyl groups, R² is selected form the group consisting of hydrogen atoms, fluorine atoms, chlorine atoms and methyl groups, and either G³ represents a group CH and G⁴ represents a nitrogen atom or G³ represents a nitrogen atom and G⁴ represents a group CH.

More preferably, both groups G¹ represent C(R^{c}) groups, G² represents C(R^{d}) group, R^{a} is a fluorine atom, R^{b} is selected from the group consisting of hydrogen atoms and fluorine atoms and R¹ is selected from the group consisting of hydrogen, bromine and fluorine atoms, methyl, ethyl and cyclopropyl groups. Preferably R^{c} is a hydrogen atom, R^{d} is selected from the group consisting of C₁₋₃ alkoxy and C₃₋₄ cycloalkoxy groups and R² is a hydrogen atom. Particularly preferred are the compounds wherein G³ represents a nitrogen atom, G⁴ represents a group CH and R^{b} is a fluorine atom and the compounds wherein G³ represents a group CH, G⁴ represents a nitrogen atom and both R^{a} and R^{b} are fluorine atoms.

Particularly preferred are the compounds wherein both groups G¹ represent C(R^{c}) groups, G² represents a C(R^{d}) group, preferably G² is a group selected from C(OH), C(OMe) and C(OEt); R^{a} is selected from the group consisting of fluorine atoms and 2,2,2-trifluoroethoxy groups, R^{b} is selected from the group consisting of hydrogen atoms and fluorine atoms, R¹ is selected from the group consisting of hydrogen, bromine and fluorine atoms, methyl, ethyl and cyclopropyl groups, G³ represents a group CH and G⁴ represents a nitrogen atom.

Preferably, when the amino derivative of formula (I) A-NH-B is the amino(iso)nicotinic acid derivative of formula (I-a), it is selected from the group consisting of:
2-(3-Fluoro-3'-methoxybiphenyl-4-ylamino)nicotinic acid;
2-(3'-Ethoxy-3-fluorobiphenyl-4-ylamino)nicotinic acid;
2-(3-Fluoro-3'-(trifluoromethoxy)biphenyl-4-ylamino)nicotinic acid;
2-(3'-Ethoxy-3-(trifluoromethoxy)biphenyl-4-ylamino)nicotinic acid;
2-(3'-Methoxy-3-(trifluoromethoxy)biphenyl-4-ylamino)nicotinic acid;
2-(2,5-Difluoro-3'-methoxybiphenyl-4-ylamino)nicotinic acid;
2-(3'-Ethoxy-2,5-difluorobiphenyl-4-ylamino)nicotinic acid;
2-(2',3-difluoro-3'-methoxybiphenyl-4-ylamino)nicotinic acid;
2-(2-Methyl-3'-(trifluoromethoxy)biphenyl-4-ylamino)nicotinic acid;
2-(3-Chloro-3'-(trifluoromethoxy)biphenyl-4-ylamino)nicotinic acid;
2-(3-Chloro-3'-ethoxybiphenyl-4-ylamino)nicotinic acid;
2-(3-Methyl-3'-(trifluoromethoxy)biphenyl-4-ylamino)nicotinic acid;
2-(3-Chloro-3'-methoxybiphenyl-4-ylamino)nicotinic acid;
2-(3'-(Difluoromethoxy)-3-fluorobiphenyl-4-ylamino)nicotinic acid;
2-(3'-Cyclobutoxy-3-fluorobiphenyl-4-ylamino)nicotinic acid;
2-(3-Fluoro-3'-(2,2,2-trifluoroethoxy)biphenyl-4-ylamino)nicotinic acid;
2-(3'-Cyclobutoxy-3,5-difluorobiphenyl-4-ylamino)nicotinic acid;
2-(3,5-Difluoro-3'-(trifluoromethoxy)biphenyl-4-ylamino)nicotinic acid;
2-(3'-Ethoxy-3,5-difluorobiphenyl-4-ylamino)nicotinic acid;
2-(3,5-Difluoro-3'-methoxybiphenyl-4-ylamino)nicotinic acid;
Lithium 3-(3'-ethoxy-3-fluorobiphenyl-4-ylamino) isonicotinate;
Lithium 3-(3-fluoro-3'-methoxybiphenyl-4-ylamino)isonicotinate;
Lithium 3-(3'-methoxy-3-(trifluoromethoxy)biphenyl-4-ylamino)isonicotinate;
Lithium 3-(3-fluoro-3'-(trifluoromethoxy)biphenyl-4-ylamino)isonicotinate;
2-(3'-Ethoxybiphenyl-4-ylamino)nicotinic acid;
2-(5-Fluoro-2-methyl-3'-(trifluoromethoxy)biphenyl-4-ylamino)nicotinic acid;
2-(2',3-Difluoro-5'-isopropoxybiphenyl-4-ylamino)nicotinic acid;
2-(3-Fluoro-3'-methoxybiphenyl-4-ylamino)-5-methylnicotinic acid;
2-(3,5 -Difluoro-3'-hydroxybiphenyl-4-ylamino)nicotinic acid;
5-Bromo-2-(3-fluoro-3'-methoxybiphenyl-4-ylamino)nicotinic acid;
5-Bromo-2-(3,5-difluoro-3'-methoxybiphenyl-4-ylamino)nicotinic acid;
5-Bromo-2-(3-fluoro-3'-(trifluoromethoxy)biphenyl-4-ylamino)nicotinic acid;
2-(3-Fluoro-3'-(trifluoromethoxy)biphenyl-4-ylamino)-5-methylnicotinic acid;
5-Cyclopropyl-2-(3-fluoro-3'-methoxybiphenyl-4-ylamino)nicotinic acid;
2-(3,5-Difluoro-3'-methoxybiphenyl-4-ylamino)-5-methylnicotinic acid;
2-(3'-Ethoxy-5-fluoro-2-methylbiphenyl-4-ylamino)nicotinic acid;
2-(5-Fluoro-3'-methoxy-2-methylbiphenyl-4-ylamino)nicotinic acid;
2-(3'-ethoxy-3,5-difluorobiphenyl-4-ylamino)-5-methylnicotinic acid;
5-cyclopropyl-2-(3'-ethoxy-3,5-difluorobiphenyl-4-ylamino)nicotinic acid;
2-(3,5-difluoro-3'-methoxybiphenyl-4-ylamino)-5-ethylnicotinic acid;
5-bromo-2-(3'-ethoxy-2,5-difluorobiphenyl-4-ylamino)nicotinic acid;
5-cyclopropyl-2-(3'-ethoxy-2,5-difluorobiphenyl-4-ylamino)nicotinic acid;
2-(5-fluoro-3'-methoxy-2-methylbiphenyl-4-ylamino)-5-methylnicotinic acid;
5-cyclopropyl-2-(5-fluoro-3'-methoxy-2-methylbiphenyl-4-ylamino)nicotinic acid;
2-(2',3,5-trifluoro-3'-methoxybiphenyl-4-ylamino)nicotinic acid;
2-(2'-chloro-3,5-difluorobiphenyl-4-ylamino)nicotinic acid;
2-(3'-cyclopropoxy-3,5-difluorobiphenyl-4-ylamino)nicotinic acid;
2-(3,5-difluoro-2-methylbiphenyl-4-ylamino)nicotinic acid;
5-cyclopropyl-2-(2,5-difluoro-3'-methoxybiphenyl-4-ylamino)nicotinic acid;
2-(3'-cyclopropoxy-3,5-difluorobiphenyl-4-ylamino)-5-cyclopropylnicotinic acid;
5-chloro-2-(3,5-difluoro-3'-methoxybiphenyl-4-ylamino)nicotinic acid;
5-cyclopropyl-2-(3,5-difluoro-3'-(trifluoromethoxy)biphenyl-4-ylamino)nicotinic acid;
2-(2,3,5-trifluoro-3'-methoxybiphenyl-4-ylamino)nicotinic acid;
2-(2'-chloro-3,5-difluorobiphenyl-4-ylamino)-5-cyclopropylnicotinic acid;
2-(3,5-difluoro-3'-methoxy-2-methylbiphenyl-4-ylamino)nicotinic acid;
2-(3,5-difluoro-2-methyl-3'-(trifluoromethoxy)biphenyl-4-ylamino)nicotinic acid;
2-(2'-chloro-3,5-difluoro-2-methylbiphenyl-4-ylamino)nicotinic acid;
5-chloro-2-(3,5-difluorobiphenyl-4-ylamino)nicotinic acid;
5-chloro-2-(2'-chloro-3,5-difluorobiphenyl-4-ylamino)nicotinic acid;
2-(2,3,5,6-tetrafluoro-3'-methoxybiphenyl-4-ylamino)nicotinic acid;
2-(3,5-difluoro-2'-methylbiphenyl-4-ylamino)nicotinic acid;
3-(3'-cyclopropoxy-3-fluorobiphenyl-4-ylamino)isonicotinic acid;
or a pharmaceutically acceptable salt, or solvate, or N-oxide, or stereoisomer or deuterated derivate thereof.

More preferably, when the amino derivative of formula (I) A-NH-B is the amino(iso)nicotinic acid derivative of formula (I-a), it is selected from the group consisting of:
2-(3'-ethoxy-3-(trifluoromethoxy)biphenyl-4-ylamino)nicotinic acid;
2-(3'-methoxy-3-(trifluoromethoxy)biphenyl-4-ylamino)nicotinic acid;
2-(3'-ethoxy-3,5-difluorobiphenyl-4-ylamino)nicotinic acid;
2-(3,5-difluoro-3'-methoxybiphenyl-4-ylamino)nicotinic acid;
lithium 3-(3'-ethoxy-3-fluorobiphenyl-4-ylamino)isonicotinate:
lithium 3-(3-fluoro-3'-methoxybiphenyl-4-ylamino)isonicotinate;
lithium 3-(3'-methoxy-3-(trifluoromethoxy)biphenyl-4-ylamino)isonicotinate;
2-(3-fluoro-3'-methoxybiphenyl-4-ylamino)-5-methylnicotinic acid;
5-bromo-2-(3,5-difluoro-3'-methoxybiphenyl-4-ylamino)nicotinic acid;
5-cyclopropyl-2-(3-fluoro-3'-methoxybiphenyl-4-ylamino)nicotinic acid;
2-(3,5-difluoro-3'-methoxybiphenyl-4-ylamino)-5-methylnicotinic acid;
2-(3,5-difluoro-3'-methoxybiphenyl-4-ylamino)-5-ethylnicotinic acid;
2-(2'-chloro-3,5-difluorobiphenyl-4-ylamino)nicotinic acid;
2-(3'-cyclopropoxy-3,5-difluorobiphenyl-4-ylamino)nicotinic acid;
2-(3,5-difluoro-2-methylbiphenyl-4-ylamino)nicotinic acid;
5-cyclopropyl-2-(2,5-difluoro-3'-methoxybiphenyl-4-ylamino)nicotinic acid;
5-cyclopropyl-2-(3,5-difluoro-3'-(trifluoromethoxy)biphenyl-4-ylamino)nicotinic acid;
2-(2'-chloro-3,5-difluorobiphenyl-4-ylamino)-5-cyclopropylnicotinic acid;
2-(2,3,5,6-tetrafluoro-3'-methoxybiphenyl-4-ylamino)nicotinic acid;
and their pharmaceutically acceptable salts, their solvates, their N-oxides, their stereoisomers or their deuterated derivates thereof.

Even more preferably, when the amino derivative of formula (I) A-NH-B is the amino(iso)nicotinic acid derivative of formula (I-a), it is selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid, 2-(3'-cyclopropoxy-3,5-difluorobiphenyl-4-ylamino)nicotinic acid, 2-(3,5-difluoro-2-methylbiphenyl-4-ylamino)nicotinic acid and 5-cyclopropyl-2-(3,5-difluoro-3'-(trifluoromethoxy)biphenyl-4-ylamino)nicotinic acid, and their pharmaceutically acceptable salts, their solvates, their N-oxides, their stereoisomers or their deuterated derivates thereof.

Most preferably, when the amino derivative of formula (I) A-NH-B is the amino(iso)nicotinic acid derivative of formula (I-a), it is selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid and 2-(3,5-difluoro-2-methylbiphenyl-4-ylamino)nicotinic acid,
and their pharmaceutically acceptable salts, their solvates, their N-oxides, their stereoisomers or their deuterated derivates thereof.

Preferred pharmaceutically acceptable salts of the amino(iso)nicotinic acid derivative of formula (I-a) are formed with the pharmaceutically acceptable bases selected from L-arginine, meglumine and tromethamine, being meglumine and tromethamine particularly preferred.

Of particular interest are the salts of the amino(iso)nicotinic acid derivative of formula (I-a) selected from the group consisting of 2-(3'-ethoxy-3-(trifluoromethoxy)biphenyl-4-ylamino)nicotinic acid, meglumine salt; 2-(3,5-difluoro-3'-methoxybiphenyl-4-ylamino)nicotinic acid, meglumine salt; 2-(3,5-difluoro-3'-methoxybiphenyl-4-ylamino)nicotinic acid, tromethamine salt; 2-(3,5-difluoro-3'-methoxybiphenyl-4-ylamino)nicotinic acid, L-arginine salt; 2-(3,5-difluoro-2-methylbiphenyl-4-ylamino)nicotinic acid, meglumine salt; and 2-(3,5-difluoro-2-methylbiphenyl-4-ylamino)nicotinic acid, tromethamine salt.

### The azabiphenylaminobenzoic acid derivative of formula (I-b)

In a further embodiment, the amino derivative of formula (I) A-NH-B is an azabiphenylaminobenzoic acid derivative of formula (I-b) 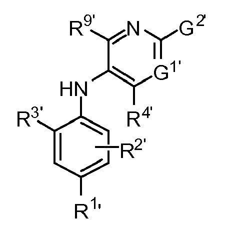 Formula (I-b)
wherein
R^{1'} is selected from the group consisting of hydrogen atoms, halogen atoms, C₁₋₄ alkyl, C₃₋₄ cycloalkyl, -CF₃ and -OCF₃,
R^{2'} is selected from the group consisting of hydrogen atoms, halogen atoms and C₁₋₄ alkyl groups,
R^{3'} is selected from the group consisting of -COOR^{5'}, -CONHR^{5'}, tetrazolyl, -SO₂NHR^{5'} and -CONHSO₂R^{5'} groups, wherein R^{5'} is selected from the group consisting of a hydrogen atom and linear or branched C₁₋₄ alkyl groups,
R^{4'} is selected from the group consisting of a hydrogen atom and a C₁₋₄ alkyl group,
R^{9'} is selected from the group consisting of a hydrogen atom and a phenyl group,
G^{1'} represents a group selected from N and CR^{6'} wherein R^{6'} is selected from the group consisting of hydrogen atoms, halogen atoms, C₁₋₄ alkyl, C₃₋₄ cycloalkyl, C₁₋₄ alkoxy, -CF₃, -OCF₃, monocyclic N-containing C₅₋₇ heteroaryl, monocyclic N-containing C₃₋₇ heterocyclyl groups and C₆₋₁₀ aryl groups which C₆₋₁₀ aryl groups are optionally substituted with one or more substituents selected from halogen atoms and C₁₋₄ alkyl groups,
G^{2'} represents a group selected from:
   - a hydrogen atom, a hydroxy group, a halogen atom, a C₃₋₄ cycloalkyl group, a C₁₋₄ alkoxy group and -NR^{a}'R^{b'}, wherein
      R^{a'} represents a C₁₋₄ alkyl group and R^{b'} is selected from a group consisting of C₁₋₄ alkyl group and C₁₋₄ alkoxy-C₁₋₄ alkyl group, or
      R^{a'} and R^{b'} together with the nitrogen atom to which they are attached form a saturated 6 to 8 membered heterocyclic ring optionally containing one oxygen atom as an additional heteroatom,
   - a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄cycloalkoxy, -CF₃, -OCF₃, and -CONR^{7'}R^{8'}, wherein R^{7'} and R^{8'} are independently selected from hydrogen atom, linear or branched C₁₋₄ alkyl groups, C₃₋₇cycloalkyl groups, or R^{7'} and R^{8'} together with the nitrogen atom to which they are attached form a group of formula 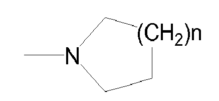 wherein n is an integer from 0 to 3,
      and
   - a phenyl group which is optionally substituted by one or more substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR^{7'}R^{8'}, oxadiazolyl, triazolyl, pyrazolyl and imidazolyl groups, which oxadiazolyl, triazolyl, pyrazolyl and imidazolyl groups are optionally substituted by C₁₋₄ alkyl or C₃₋₇ cycloalkyl groups and wherein R^{7'} and R^{8'} are independently selected from hydrogen atom, linear or branched C₁₋₄ alkyl groups, C₃₋₇ cycloalkyl groups, or R^{7'} and R^{3'} together with the nitrogen atom to which they are attached form a group of formula 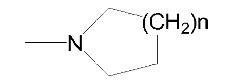 wherein n is an integer from 0 to 3,
      or, when G^{1'} represents CR^{6'}, G^{2'} together with R^{6'} forms a non-aromatic C₅₋₁₀ carbocyclic group or a C₆₋₁₀ aryl group,
   or a pharmaceutically acceptable salt, or solvate, or N-oxide, or stereoisomer or deuterated derivate thereof.

The azabiphenylaminobenzoic acid derivative of formula (I-b) and methods for their preparation are described in the International Patent Application No. W02009/021696. As used herein, the term aryl radical embraces C₆-C₁₀ monocyclic or polycyclic aryl radical such as phenyl, naphthyl, anthranyl and phenanthryl. Phenyl is preferred.

A said optionally substituted aryl radical is typically unsubstituted or substituted with 1, 2 or 3 substituents which may be the same or different. The substituents are preferably selected from halogen atoms, preferably fluorine atoms, hydroxy groups, alkoxycarbonyl groups in which the alkyl moiety has from 1 to 4 carbon atoms, hydroxycarbonyl groups, carbamoyl groups, nitro groups, cyano groups, C₁-C₄ alkyl groups, C₁-C₄ alkoxy groups and C₁-C₄ hydroxyalkyl groups. When an aryl radical carries 2 or more substituents, the substituents may be the same or different. Unless otherwise specified, the substituents on an aryl group are typically themselves unsubstituted.

As used herein, the terms heteroaryl and heteroaromatic ring are used interchangeably and typically embrace a 5- to 14- membered ring system, preferably a 5- to 10- membered ring system, comprising at least one heteroaromatic ring and containing at least one heteroatom selected from O, S and N. A heteroaryl radical may be a single ring (monocyclic) or two or more fused rings (polycyclic) wherein at least one ring contains a heteroatom.

As used herein, the term heterocyclyl radical embraces typically a non-aromatic, saturated or unsaturated C₃-C₁₀ carbocyclic ring system, such as a 5, 6 or 7 membered radical, in which one or more, for example 1, 2, 3 or 4 of the carbon atoms preferably 1 or 2 of the carbon atoms are replaced by a heteroatom selected from N, O and S. Saturated heterocyclyl radicals are preferred.

Typically, R^{1'} is selected from the group consisting of hydrogen atoms, fluorine atoms, chlorine atoms, bromine atoms, C₁₋₄ alkyl, C₃₋₄ cycloalkyl and -CF₃ groups.

Typically R^{2'} is selected from the group consisting of hydrogen atoms, halogen atoms and methyl groups.

Typically, G^{1'} is selected from the group consisting of nitrogen atoms, CCI, CF, CH, C(CH₃), C(cyclopropyl), C(phenyl) and C(CF₃) groups.

Typically G^{2'} represents a group selected from:
- a hydrogen atom, a halogen atom, a C₃₋₄ cycloalkyl group, a C₁₋₂ alkoxy group and -NR^{a'}R^{b'}, wherein
   R^{a} represents a C₁₋₂ alkyl group and R^{b'} is selected from the group consisting of C₁₋₂ alkyl groups and C₁₋₂ alkoxy-C₁₋₂ alkyl groups, or
   R^{a'} and R^{b'} together with the nitrogen atom to which they are attached form a saturated 6 or 7 membered heterocyclic ring optionally containing one oxygen atom as an additional heteroatom,
- a monocyclic or bicyclic 5 to 10 membered heteroaromatic ring containing one or two nitrogen atoms which is optionally substituted by one or more substituents selected from halogen atoms and C₁₋₄ alkyl groups,
   and
- a phenyl group which is optionally substituted by one, two or three substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR^{7'}R^{8'} and oxadiazolyl groups, which oxadiazolyl groups are optionally substituted by a C₁₋₄ alkyl or a C₃₋₇ cycloalkyl group and wherein R^{7'} and R^{8'} are independently selected from hydrogen atoms, linear or branched C₁₋₄ alkyl groups, C₃₋₄ cycloalkyl groups, or R^{7'} and R^{8'} together with the nitrogen atom to which they are attached form a group of formula 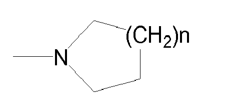 wherein n is 1 or 2,
   or, when G^{1'} represents CR^{6'}, G^{2'} together with R^{6'} forms a non-aromatic C₆ carbocyclic group or a phenyl group.

More typically G^{2'} represents a group selected from:
- a hydrogen atom, a fluorine atom, a cyclopropyl group, a methoxy group, -NMeEt, -NEt₂, -N(Me)-(CH₂)₂-O-CH₃, 6-morpholinyl, azepan-1-yl and piperidin-1-yl,
- a pyridinyl, pyrimidinyl, quinolinyl or pyrazinyl ring optionally substituted with one or two substituents selected from Me and F
   and
- a phenyl group which is optionally substituted by one, two or three substituents selected from fluorine, chlorine, methyl, hydroxyl, methoxy, ethoxy, isopropyloxy, cyclopropyl, cyclopropyloxy, cyano, -CF₃, -OCF₃ ,oxadiazolyl and -CONR^{7'}R^{8'} groups, which oxadiazolyl groups are optionally substituted by a methyl group and wherein R^{7'} and R^{8'} are independently selected from hydrogen atom, methyl group, isopropyl group, cyclopropyl group, or R^{7'} and R^{8'} together with the nitrogen atom to which they are attached form a group of formula 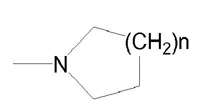 wherein n is 1,
   or, when G^{1'} represents CR^{6'}, G^{2'} together with R^{6'} forms a non-aromatic C₆ carbocyclic group or a phenyl group.

Even more typically, G^{2'} represents a group selected from methoxy group, cyclopropyl group and optionally substituted phenyl, pyridyl, quinolynyl, pyrimidinyl and pyrazinyl groups.

In one embodiment of the present invention, R^{1'} is selected from the group consisting of hydrogen atoms, halogen atoms, C₁₋₄ alkyl, C₃₋₄ cycloalkyl, -CF₃ and -OCF₃,
R^{2'} is selected from the group consisting of hydrogen atoms, halogen atoms and C₁₋₄ alkyl group,
R^{3'} is selected from the group consisting of -COOR^{5'}, -CONHR^{5'}, tetrazolyl, -SO₂NHR^{5'} and -CONHSO₂R^{5'} groups, wherein R^{5'} is selected from the group consisting of a hydrogen atom and lineal or branched C₁₋₄ alkyl groups,
R^{4'} is selected from the group consisting of a hydrogen atom and a C₁₋₄ alkyl group
R^{9'} represents a hydrogen atom,
G^{1'} represents a group selected from N and CR^{6'} wherein R^{6'} is selected from the group consisting of hydrogen atoms, halogen atoms, C₁₋₄ alkyl, C₃₋₄ cycloalkyl, C₁₋₄ alkoxy, - CF₃, -OCF₃, monocyclic N-containing C₅₋₇ heteroaryl, monocyclic N-containing C₃₋₇ heterocyclyl groups and a C₆₋₁₀ aryl group which is optionally substituted with one or more substituents selected from halogen atoms and a C₁₋₄ alkyl group,
and G^{2'} represents a group selected from:
   - a monocyclic or bicyclic 5 to 10 membered heteroaromatic ring containing a nitrogen atom which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR^{7'}R^{8'}, wherein R^{7'} and R^{8'} are independently selected from hydrogen atom, lineal or branched C₁₋₄ alkyl group, C₃₋₇ cycloalkyl group, or R^{7'} and R^{8'} together with the nitrogen atom to which they are attached form a group of formula 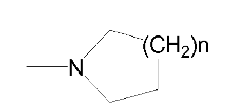 wherein n is an integer from 0 to 3,
      and
   - a phenyl group which is optionally substituted by one or more substituents selected from halogen atoms, C₁₋₄ alkyl, C₁₋₄ alkoxy, C₃₋₄cycloalkyl, C₃₋₄ cycloalkoxy, -CF₃, -OCF₃, -CONR^{7'}R^{8'}, oxadiazolyl, triazolyl, pyrazolyl and imidazolyl groups, wherein the oxadiazolyl, triazolyl, pyrazolyl and imidazolyl groups are optionally substituted by C₁₋₄ alkyl or C₃₋₇ cycloalkyl group and wherein R^{7'} and R^{8'} are independently selected from hydrogen atom, lineal or branched C₁₋₄ alkyl group, a C₃₋₇ cycloalkyl group, or R^{7'} and R^{8'} together with the nitrogen atom to which they are attached form a group of formula 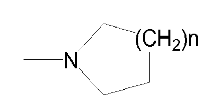 wherein n is an integer from 0 to 3.

In another embodiment of the present invention, R^{9'} represents a hydrogen atom, and G^{2'} represents a group selected from:
- a monocyclic or bicyclic 5 to 10 membered heteroaromatic ring containing a nitrogen atom which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR^{7'}R^{3'}, wherein R^{7'} and R^{8'} are independently selected from hydrogen atom, lineal or branched C₁₋₄ alkyl group, C₃₋₇cycloalkyl group, or R^{7'} and R^{8'} together with the nitrogen atom to which they are attached form a group of formula 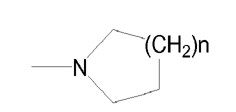 wherein n is an integer from 0 to 3,
   and
- a phenyl group which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, -CONR^{7'}R^{8'}, oxadiazolyl, triazolyl, pyrazolyl and imidazolyl groups, wherein the oxadiazolyl, triazolyl, pyrazolyl and imidazolyl groups are optionally substituted by C₁₋₄ alkyl or C₃₋₇ cycloalkyl group and wherein R^{7'} and R^{8'} are independently selected from hydrogen atom, lineal or branched C₁₋₄ alkyl group, a C₃₋₇ cycloalkyl group, or R^{7'} and R^{3'} together with the nitrogen atom to which they are attached form a group of formula 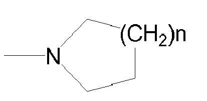 wherein n is an integer from 0 to 3.

Typically, R^{1'} is selected from the group consisting of C₁₋₄ alkyl, C₃₋₄ cycloalkyl and - CF₃, preferably methyl and cyclopropyl group, more preferably a cyclopropyl group.

Typically, R^{2'} is selected from a hydrogen or halogen atom, preferably a hydrogen atom.

Typically, R^{3'} is selected from COOR⁵, -CONHR⁵ and tetrazolyl group; preferably R^{3'} is a COOH group.

Typically, R^{4'} represents a hydrogen atom or a methyl group, preferably a hydrogen atom.

Typically, R^{9'} represents a hydrogen atom.

Typically, G^{1'} represents a group selected from N, CH, C(CH₃), C(cyclopropyl), C(phenyl) or C(CF₃) groups.

Typically, G^{2'} is selected from the group consisting of a methoxy group, a cyclopropyl group and optionally substituted phenyl, pyridyl, quinolynyl, pyrimidinyl and pyrazinyl groups, more preferably, G^{2'} is selected from the group consisting of optionally substituted phenyl, pyridyl, quinolynyl, pyrimidinyl and pyrazinyl groups, most preferably selected from optionally substituted phenyl, 4-pyridyl, 5-quinolynyl and 2-pyrazinyl groups.

Preferred compounds of formula (I-b) are those in which R^{1'} is selected from a hydrogen atom, halogen atom, a methyl group , a CF3 group and a cyclopropyl group, R^{2'} represents a hydrogen atom or a methyl group, R^{3'} is a COOR^{5'} group wherein R^{5'} is selected from the group consisting of a hydrogen atom a methyl group, an ethyl group and a tert-butyl group, R^{4'} represents a hydrogen atom, a fluorine atom or a methyl group, R^{9'} represents a hydrogen atom or a phenyl group, G^{1'} is selected from nitrogen atoms and CR^{6'} wherein R^{6'} represents a hydrogen atom, a fluorine atom, a chlorine atom, a methyl group, a trifluoromethyl group, a cyclopropyl group and a phenyl group, and G^{2'} represents a group selected from:
- a hydrogen atom, a hydroxy group, a fluorine atom, a cyclopropyl group, a methoxy group and -NR^{a'}R^{b'}, wherein
   R^{a} represents a C₁₋₂ alkyl group and R^{b'} is selected from a group consisting of C₁₋₂ alkyl group and C₁₋₂ alkoxy-C₁₋₂ alkyl group, or R^{a'} and R^{b'} together with the nitrogen atom to which they are attached form a saturated 6 to 7 membered heterocyclic ring optionally containing one oxygen atom as an additional heteroatom;
- a monocyclic or bicyclic 5 to 10 membered heteroaromatic ring containing one or two nitrogen atoms which is optionally substituted by one or two halogen atoms, and
   -CONR^{7'}R^{8'}, wherein R^{7'} and R^{8'} are independently selected from a hydrogen atom, a C₁₋₂ alkyl group, and a cyclopropyl group, or R^{7'} and R^{8'} together with the nitrogen atom to which they are attached form a group of formula 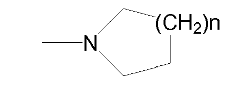 wherein n is 1;
   and
- a phenyl group which is optionally substituted by one, two or three substituents selected from fluorine atoms, chlorine atoms, hydroxy, C₁₋₃ alkoxy, cyclopropyl, cyclopropoxy, cyano, -CF₃, -OCF₃,
   -CONR₇R₈ and oxadiazolyl groups, wherein the oxadiazolyl groups are optionally substituted by a methyl group and wherein R₇ and R₈ are as defined above;
or, when G^{1'} represents CR^{6'}, G^{2'} together with R^{6'} form a non-aromatic C₆ carbocyclic group or a phenyl group.

In yet another embodiment of the present invention, R^{1'} is selected from a methyl or cyclopropyl group, R^{2'} represents a hydrogen atom, R^{3'} is a COOH group, R^{4'} represents a hydrogen atom or a methyl group, G^{1'} is selected from N, CH, C(CH₃), C(cyclopropyl), C(phenyl) and C(CF₃) groups, and G^{2'} represents a group selected from the group consisting of optionally substituted phenyl, 4-pyridyl, 5-quinolynyl and 2-pyrazinyl groups, more preferably R^{9'} represent a hydrogen group.

In yet another embodiment of the present invention, R^{1'} is selected from a methyl or cyclopropyl group, R^{2'} represents a hydrogen atom, R^{3'} is a COOH group, R^{4'} represents a hydrogen atom, G^{1'} is selected from nitrogen atoms and CH, C(CH₃) and C(CF₃) groups and G^{2'} represents a phenyl group optionally substituted with one or two substituents selected from chloro, fluoro, methoxy, ethoxy, isopropoxy, trifluoromethoxy and -CONR^{7'}R^{8'}, wherein R^{7'} is hydrogen and R^{8'} is cyclopropyl or R^{7'} and R^{8'} together with the nitrogen atom to which they are attached form a group of formula 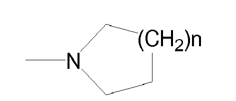 wherein n is 1.

Preferably, when the amine derivative of formula (I) A-NH-B is the azabiphenylaminobenzoic acid derivative of formula (I-b), it is selected from the group consisting of:
5-cyclopropyl-2-(2-phenylpyrimidin-5-ylamino)benzoic acid;
2-(6-Cyclopropyl-5-methylpyridin-3-ylamino)-5-methylbenzoic acid;
5-(2-Carboxy-4-cyclopropylphenylamino)-3-methyl-2-phenylpyridine 1-oxide;
5-Methyl-2-(6-(3-(trifluoromethyl)phenyl)pyridin-3-ylamino)benzoic acid;
5-cyclopropyl-2-(6-hydroxy-5-phenylpyridin-3-ylamino)benzoic acid;
5-cyclopropyl-2-(2-(2,6-difluoro-4-hydroxyphenyl)pyrimidin-5-ylamino)benzoic acid;
5-Cyclopropyl-2-(6-methoxy-5-phenylpyridin-3-ylamino) benzoic acid;
2-(5-Fluoro-6-phenylpyridin-3-ylamino)-5-methylbenzoic acid;
2-(6-(Ethyl(methyl)amino)-5-methylpyridin-3-ylamino)-5-methylbenzoic acid;
5-Cyclopropyl-2-(3'-fluoro-2,4'-bipyridin-5-ylamino)benzoic acid;
2-(6-(Diethylamino)-5-methylpyridin-3-ylamino)-5-methylbenzoic acid;
2-(6-((2-Methoxyethyl)(methyl)amino)-5-methylpyridin-3-ylamino)-5-methylbenzoic acid;
2-(5-Chloro-6-phenylpyridin-3-ylamino)-5-methylbenzoic acid;
5-Cyclopropyl-2-(2-(2-cyclopropylphenyl)pyrimidin-5-ylamino)benzoic acid;
5-cyclopropyl-2-(5-phenylpyridin-3-ylamino) benzoic acid;
5-methyl-2-(quinolin-3-ylamino)benzoic acid;
5-methyl-2-(5,6,7,8-tetrahydroquinolin-3-ylamino)benzoic acid;
2-(5-Chloro-2-phenylpyridin-3-ylamino)-5-methylbenzoic acid;
5-Cyclopropyl-2-(5,6-diphenylpyridin-3-ylamino)benzoic acid;
5-cyclopropyl-2-(2-(2,6-difluorophenyl)pyrimidin-5-ylamino)benzoic acid;
5-Cyclopropyl-2-(5-methylpyridin-3-ylamino) benzoic acid;
2-(2-(3-Cyclopropoxyphenyl)pyrimidin-5-ylamino)-5-cyclopropylbenzoic acid;
5-Methyl-2-(6-morpholinopyridin-3-ylamino)benzoic acid;
5-Methyl-2-(5-methyl-6-morpholinopyridin-3-ylamino)benzoic acid;
5-cyclopropyl-2-(6-cyclopropyl-5-phenylpyridin-3-ylamino) benzoic acid;
2-(6-(2-Cyclopropylphenyl)-5-methylpyridin-3-ylamino)-5-methylbenzoic acid;
2-(6-(2-Cyanophenyl)-5-methylpyridin-3-ylamino)-5-methylbenzoic acid;
2-(2-(3-Chlorophenyl)pyrimidin-5-ylamino)-5-cyclopropylbenzoic acid;
5-Methyl-2-(6-phenyl-5-(trifluoromethyl)pyridin-3-ylamino)benzoic acid;
5-Methyl-2-(5-methyl-6-(piperidin-1-yl)pyridin-3-ylamino)benzoic acid;
2-(6-(Azepan-1-yl)-5-methylpyridin-3-ylamino)-5-methylbenzoic acid;
2-(6-(3-Methoxyphenyl)-5-phenylpyridin-3-ylamino)-5-methylbenzoic acid;
2-(2,3'-bipyridin-5-ylamino)-5-cyclopropylbenzoic acid;
2-(3'-chloro-2,4'-bipyridin-5-ylamino)-5-methylbenzoic acid;
5-Methyl-2-(3-methyl-2,2'-bipyridin-5-ylamino)benzoic acid;
2-(5,6-Difluoropyridin-3-ylamino)-5-methylbenzoic acid;
2-(6-(3-Methoxyphenyl)pyridin-3-ylamino)benzoic acid;
2-(6-(3-Ethoxyphenyl)pyridin-3-ylamino)benzoic acid;
2-(6-(3-Ethoxyphenyl)pyridin-3-ylamino)-5-fluorobenzoic acid;
2-(6-(3-Ethoxyphenyl)-5-methylpyridin-3-ylamino)benzoic acid;
2-(6-(3-Ethoxyphenyl)pyridin-3-ylamino)-5-methylbenzoic acid;
2-(6-(3-Ethoxyphenyl)-5-methylpyridin-3-ylamino)-5-methylbenzoic acid;
2-(6-(3-Ethoxy-2-fluorophenyl)pyridin-3-ylamino)benzoic acid;
2-(6-(3-Ethoxyphenyl)-4-methylpyridin-3-ylamino)-5-methylbenzoic acid;
2-(6-(3-Ethoxyphenyl)-4-methylpyridin-3-ylamino)benzoic acid;
5-Bromo-2-(6-(3-ethoxyphenyl)pyridin-3-ylamino)benzoic acid;
5-Chloro-2-(6-(3-ethoxyphenyl)pyridin-3-ylamino)benzoic acid;
2-(6-(5-Ethoxy-2-fluorophenyl)pyridin-3-ylamino)benzoic acid;
2-(6-(3-Ethoxyphenyl)-5-methylpyridin-3-ylamino)-5-(trifluoromethyl)benzoic acid;
2-(6-(3-Methoxyphenyl)-5-methylpyridin-3-ylamino)-5-(trifluoromethyl)benzoic acid;
2-(6-(3-Methoxyphenyl)-5-methylpyridin-3-ylamino)-5-methylbenzoic acid;
2-(6-(3-Methoxyphenyl)-5-methylpyridin-3-ylamino)-6-methylbenzoic acid;
5-Fluoro-2-(6-(3-methoxyphenyl)-5-methylpyridin-3-ylamino)benzoic acid;
2-(6-(5-Ethoxy-2-fluorophenyl)pyridin-3-ylamino)-5-methylbenzoic acid;
2-(6-(2-Fluoro-5-methoxyphenyl)-5-methylpyridin-3-ylamino)-5-methylbenzoic acid;
Ethyl 2-(6-(2-fluoro-5-methoxyphenyl)-5-methylpyridin-3-ylamino)-5-methylbenzoate;
2-(6-(2-Fluorophenyl)pyridin-3-ylamino)-5-methylbenzoic acid;
2-(6-(3-Methoxyphenyl)-5-phenylpyridin-3-ylamino)-5-methylbenzoic acid;
Ethyl 2-(6-(3-methoxyphenyl)-5-phenylpyridin-3-ylamino)-5-methylbenzoate;
5-Methyl-2-(5-methyl-6-phenylpyridin-3-ylamino)benzoic acid;
Ethyl 5-methyl-2-(5-methyl-6-phenylpyridin-3-ylamino)benzoate;
5-Methyl-2-(5-methyl-6-(3-(trifluoromethoxy)phenyl)pyridin-3-ylamino)benzoic acid;
Ethyl 5-methyl-2-(5-methyl-6-(3-(trifluoromethoxy)phenyl)pyridin-3-ylamino)benzoate;
2-(5-Cyclopropyl-6-(3-methoxyphenyl)pyridin-3-ylamino)-5-methylbenzoic acid;
Ethyl 2-(5-cyclopropyl-6-(3-methoxyphenyl)pyridin-3-ylamino)-5-methylbenzoate;
2-(6-(2-Fluoro-5-isopropoxyphenyl)pyridin-3-ylamino)-5-methylbenzoic acid;
2-(6-(3-Isopropoxyphenyl)-5-methylpyridin-3-ylamino)-5-methylbenzoic acid;
Ethyl 2-(6-(3-isopropoxyphenyl)-5-methylpyridin-3-ylamino)-5-methylbenzoate;
2-(6-(3-Cyclopropoxyphenyl)-5-methylpyridin-3-ylamino)-5-methylbenzoic acid;
*tert*-Butyl 2-(6-(3-cyclopropoxyphenyl)-5-methylpyridin-3-ylamino)-5-methylbenzoate;
2-(6-(2-Chlorophenyl)-5-methylpyridin-3-ylamino)-5-methylbenzoic acid;
*tert-*Butyl 2-(6-(2-chlorophenyl)-5-methylpyridin-3-ylamino)-5-methylbenzoate; 2-(6-(3-Carbamoylphenyl)-5-methylpyridin-3-ylamino)-5-methylbenzoic acid;
Ethyl 2-(6-(3-carbamoylphenyl)-5-methylpyridin-3-ylamino)-5-methylbenzoate;
2-(6-(2-Fluoro-5-methoxyphenyl)-4-methylpyridin-3-ylamino)-5-methylbenzoic acid;
Ethyl 2-(6-(2-fluoro-5-methoxyphenyl)-4-methylpyridin-3-ylamino)-5-methylbenzoate;
2-(6-(3-Methoxyphenyl)-5-(trifluoromethyl)pyridin-3-ylamino)-5-methylbenzoic acid;
Ethyl 2-(6-(3-methoxyphenyl)-5-(trifluoromethyl)pyridin-3-ylamino)-5-methylbenzoate;
2-(6-(3-(Dimethylcarbamoyl)phenyl)-5-methylpyridin-3-ylamino)-5-methylbenzoic acid;
Ethyl 2-(6-(3-(dimethylcarbamoyl)phenyl)-5-methylpyridin-3-ylamino)-5-methylbenzoate;
2-(6-(3-Isopropoxyphenyl)-5-methylpyridin-3-ylamino)-3-methylbenzoic acid;
*tert*-Butyl 2-(6-(3-isopropoxyphenyl)-5-methylpyridin-3-ylamino)-3-methylbenzoate;
3-Methyl-2-(5-methyl-6-phenylpyridin-3-ylamino)benzoic acid;
*tert*-Butyl 3-methyl-2-(5-methyl-6-phenylpyridin-3-ylamino)benzoate;
2-(6-(2-Chlorophenyl)pyridin-3-ylamino)-5-methylbenzoic acid;
*tert*-Butyl 2-(6-(2-chlorophenyl)pyridin-3-ylamino)-5-methylbenzoate;
3-Fluoro-2-(6-(3-methoxyphenyl)-5-methylpyridin-3-ylamino)benzoic acid;
*tert*-Butyl 3-fluoro-2-(6-(3-methoxyphenyl)-5-methylpyridin-3-ylamino) benzoate;
5-Cyclopropyl-2-(5-methyl-6-(3-(trifluoromethoxy)phenyl)pyridin-3-ylamino)benzoic acid;
Ethyl 5-cyclopropyl-2-(5-methyl-6-(3-(trifluoromethoxy)phenyl)pyridin-3-ylamino)benzoate;
5-Cyclopropyl-2-(5-methyl-6-phenylpyridin-3-ylamino)benzoic acid;
Ethyl 5-cyclopropyl-2-(5-methyl-6-phenylpyridin-3-ylamino)benzoate;
5-Methyl-2-(5-methyl-6-(2-(trifluoromethyl)phenyl)pyridin-3-ylamino)benzoic acid;
*tert*-Butyl 5-methyl-2-(5-methyl-6-(2-(trifluoromethyl)phenyl)pyridin-3-ylamino)benzoate;
2-(6-(3-Chlorophenyl)-5-methylpyridin-3-ylamino)-5-methylbenzoic acid;
*tert-*Butyl 2-(6-(3-chlorophenyl)-5-methylpyridin-3-ylamino)-5-methylbenzoate;
2-(6-(2-Fluorophenyl)-5-methylpyridin-3-ylamino)-5-methylbenzoic acid;
*tert*-Butyl 2-(6-(2-fluorophenyl)-5-methylpyridin-3-ylamino)-5-methylbenzoate;
5-Methyl-2-(5-methyl-6-(quinolin-5-yl)pyridin-3-ylamino)benzoic acid;
*tert*-Butyl 5-methyl-2-(5-methyl-6-(quinolin-5-yl)pyridin-3-ylamino)benzoate;
2-(3'-Fluoro-3-methyl-2,4'-bipyridin-5-ylamino)-5-methylbenzoic acid;
*tert*-Butyl 2-(3'-fluoro-3-methyl-2,4'-bipyridin-5-ylamino)-5-methylbenzoate;
5-Methyl-2-(5-methyl-6-(pyrazin-2-yl)pyridin-3-ylamino)benzoic acid;
*tert*-Butyl 5-methyl-2-(5-methyl-6-(pyrazin-2-yl)pyridin-3-ylamino)benzoate;
5-Cyclopropyl-2-(6-phenyl-5-(trifluoromethyl)pyridin-3-ylamino)benzoic acid;
Ethyl 5-cyclopropyl-2-(6-phenyl-5-(trifluoromethyl)pyridin-3-ylamino)benzoate;
5-Cyclopropyl-2-(6-(3-methoxyphenyl)-5-(trifluoromethyl)pyridin-3-ylamino)benzoic acid;
Ethyl 5-cyclopropyl-2-(6-(3-methoxyphenyl)-5-(trifluoromethyl)pyridin-3-ylamino)benzoate;
5-Chloro-2-(6-(2-fluorophenyl)pyridin-3-ylamino)benzoic acid;
5-Chloro-2-(6-(2-chlorophenyl)pyridin-3-ylamino)benzoic acid;
5-Chloro-2-(6-(quinolin-5-yl)pyridin-3-ylamino)benzoic acid;
2-(6-(2-Chlorophenyl)pyridin-3-ylamino)-5-cyclopropylbenzoic acid;
Ethyl 2-(6-(2-chlorophenyl)pyridin-3-ylamino)-5-cyclopropylbenzoate;
5-Chloro-2-(6-(2-(trifluoromethyl)phenyl)pyridin-3-ylamino)benzoic acid;
5-Fluoro-2-(6-(2-(trifluoromethyl)phenyl)pyridin-3-ylamino)benzoic acid;
2-(3'-Fluoro-2,4'-bipyridin-5-ylamino)-5-methylbenzoic acid;
2-(2-(2-Fluorophenyl)pyrimidin-5-ylamino)-5-methylbenzoic acid;
tert-Butyl 2-(2-(2-fluorophenyl)pyrimidin-5-ylamino)-5-methylbenzoate;
2-(6-(2,6-Difluorophenyl)pyridin-3-ylamino)-5-methylbenzoic acid;
Ethyl 2-(6-(2,6-difluorophenyl)pyridin-3-ylamino)-5-methylbenzoate;
2-(2-(2-Chlorophenyl)pyrimidin-5-ylamino)-5-cyclopropylbenzoic acid;
Methyl 2-(2-(2-chlorophenyl)pyrimidin-5-ylamino)-5-cyclopropylbenzoate;
2-(2-(2-Chlorophenyl)pyrimidin-5-ylamino)-5-methylbenzoic acid;
*tert*-Butyl 2-(2-(2-chlorophenyl)pyrimidin-5-ylamino)-5-methylbenzoate ;
5-Methyl-2-(5-methyl-6-(3-(pyrrolidine-1-carbonyl)phenyl)pyridin-3-ylamino)benzoic acid;
2-(6-(3-(Cyclopropylcarbamoyl)phenyl)-5-methylpyridin-3-ylamino)-5-methylbenzoic acid;
5-Cyclopropyl-2-(2-(2-fluorophenyl)pyrimidin-5-ylamino)benzoic acid;
2-(2-(2-trifluoromethylphenyl)pyrimidin-5-ylamino)-5-cyclopropylbenzoic acid;
2-(2-*o*-tolylpyrimidin-5-ylamino)-5-cyclopropylbenzoic acid;
2-(2-(2-cyclopropoxyphenyl)pyrimidin-5-ylamino)-5-cyclopropylbenzoic acid;
2-(2-(2,5-difluorophenyl)pyrimidin-5-ylamino)-5-cyclopropylbenzoic acid;
2-(2-(2,3-difluorophenyl)pyrimidin-5-ylamino)-5-cyclopropylbenzoic acid;
2-(2-(2-fluoro-5-chlorophenyl)pyrimidin-5-ylamino)-5-cyclopropylbenzoic acid;
2-(2-(2-trifluoromethylphenyl)pyrimidin-5-ylamino)-5-methylbenzoic acid;
2-(2-(2-fluoro-5-trifluoromethoxyphenyl)pyrimidin-5-ylamino)-5-cyclopropylbenzoic acid;
2-(6-(2-trifluoromethylphenyl)pyridin-3-ylamino)-5-methylbenzoic acid;
2-(6-phenylpyridin-3-ylamino)-5-cyclopropylbenzoic acid;
2-(6-(2-fluorophenyl)pyridin-3-ylamino)-5-cyclopropylbenzoic acid;
2-(6-(3,5-difluoropyridin-4-yl)pyridin-3-ylamino)-5-methylbenzoic acid;
2-(6-(3-cyclopropylcarbamoylphenyl)pyridin-3-ylamino)-5-methylbenzoic acid;
2-(6-(2,4-difluorophenyl)pyridin-3-ylamino)-5-methylbenzoic acid;
2-(6-(2,5-difluorophenyl)pyridin-3-ylamino)-5-methylbenzoic acid;
2-(6-(2-fluorophenyl)pyridin-3-ylamino)-5-cyclopropyl-3-fluorobenzoic acid;
2-(6-(2,3,6-trifluorophenyl)pyridin-3-ylamino)-5-methylbenzoic acid;
2-(6-(3-(5-methyl-1,3,4-oxadiazol-2-yl)phenyl)pyridin-3-ylamino)-5-methylbenzoic acid;
2-(5-methyl-6-(pyrimidin-5-yl)pyridin-3-ylamino)-5-methylbenzoic acid;
2-(6-(2,3-difluorophenyl)pyridin-3-ylamino)-5-methylbenzoic acid;
2-(6-(5-fluoro-2-methoxyphenyl)-5-methylpyridin-3-ylamino)-5-methylbenzoic acid;
2-(6-(4-carbamoylphenyl)-5-methylpyridin-3-ylamino)-5-methylbenzoic acid;
Methyl 5-cyclopropyl-2-(2-(2-(trifluoromethyl)phenyl)pyrimidin-5-ylamino)benzoate;
Methyl 5-cyclopropyl-2-(2-o-tolylpyrimidin-5-ylamino)benzoate;
Methyl 2-(2-(2-cyclopropoxyphenyl)pyrimidin-5-ylamino)-5-cyclopropylbenzoate;
Methyl 5-cyclopropyl-2-(2-(2,5-difluorophenyl)pyrimidin-5-ylamino)benzoate;
Methyl 5-cyclopropyl-2-(2-(2,3-difluorophenyl)pyrimidin-5-ylamino)benzoate;
Methyl 2-(2-(5-chloro-2-fluorophenyl)pyrimidin-5-ylamino)-5-cyclopropylbenzoate;
tert-Butyl 5-methyl-2-(2-(2-(trifluoromethyl)phenyl)pyrimidin-5-ylamino)benzoate;
Methyl 5-cyclopropyl-2-(2-(2-fluoro-5-(trifluoromethyl)phenyl)pyrimidin-5-ylamino)benzoate;
Ethyl 2-(3',5'-difluoro-2,4'-bipyridin-5-ylamino)-5-methylbenzoate;
tert-Butyl 5-methyl-2-(6-(3-(5-methyl-1,3,4-oxadiazol-2-yl)phenyl)pyridin-3-ylamino)benzoate;
tert-Butyl 5-methyl-2-(5-methyl-6-(pyrimidin-5-yl)pyridin-3-ylamino)benzoate;
tert-Butyl 2-(6-(2,3-difluorophenyl)pyridin-3-ylamino)-5-methylbenzoate; and
tert-Butyl 2-(6-(5-fluoro-2-methoxyphenyl)-5-methylpyridin-3-ylamino)-5-methylbenzoate.
and their pharmaceutically acceptable salts, their solvates, their N-oxides, their stereoisomers or their deuterated derivates thereof.

More preferably, when the amine derivative of formula (I) A-NH-B is the azabiphenylaminobenzoic acid derivative of formula (I-b), it is selected from the group consisting of:
5-cyclopropyl-2-(2-phenylpyrimidin-5-ylamino)benzoic acid;
5-(2-carboxy-4-cyclopropylphenylamino)-3-methyl-2-phenylpyridine 1-oxide;
5-methyl-2-(6-(3-(trifluoromethyl)phenyl)pyridin-3-ylamino)benzoic acid;
2-(5-fluoro-6-phenylpyridin-3-ylamino)-5-methylbenzoic acid;
5-cyclopropyl-2-(3'-fluoro-2,4'-bipyridin-5-ylamino)benzoic acid;
2-(5-chloro-6-phenylpyridin-3-ylamino)-5-methylbenzoic acid;
5-cyclopropyl-2-(2-(2-cyclopropylphenyl)pyrimidin-5-ylamino)benzoic acid;
5-cyclopropyl-2-(5-phenylpyridin-3-ylamino) benzoic acid;
2-(5-chloro-2-phenylpyridin-3-ylamino)-5-methylbenzoic acid;
5-cyclopropyl-2-(5,6-diphenylpyridin-3-ylamino)benzoic acid;
5-cyclopropyl-2-(2-(2,6-difluorophenyl)pyrimidin-5-ylamino)benzoic acid;
5-cyclopropyl-2-((2-(2-(trifluoromethyl)phenyl)pyrimidin-5-yl)amino)benzoic acid;
5-methyl-2-((6-(2,3-difluorophenyl)pyridin-3-yl)amino)benzoic acid;
2-(2-(3-cyclopropoxyphenyl)pyrimidin-5-ylamino)-5-cyclopropylbenzoic acid;
5-methyl-2-(6-morpholinopyridin-3-ylamino)benzoic acid;
5-methyl-2-(5-methyl-6-morpholinopyridin-3-ylamino)benzoic acid;
5-cyclopropyl-2-(6-cyclopropyl-5-phenylpyridin-3-ylamino) benzoic acid;
2-(6-(2-cyclopropylphenyl)-5-methylpyridin-3-ylamino)-5-methylbenzoic acid;
2-(6-(2-Cyanophenyl)-5-methylpyridin-3-ylamino)-5-methylbenzoic acid;
2-(2-(3-chlorophenyl)pyrimidin-5-ylamino)-5-cyclopropylbenzoic acid;
5-methyl-2-(6-phenyl-5-(trifluoromethyl)pyridin-3-ylamino)benzoic acid;
5-methyl-2-(5-methyl-6-(piperidin-1-yl)pyridin-3-ylamino)benzoic acid;
2-(6-(azepan-1-yl)-5-methylpyridin-3-ylamino)-5-methylbenzoic acid;
2-(2,3'-bipyridin-5-ylamino)-5-cyclopropylbenzoic acid;
2-(3'-chloro-2,4'-bipyridin-5-ylamino)-5-methylbenzoic acid;
5-methyl-2-(3-methyl-2,2'-bipyridin-5-ylamino)benzoic acid;
2-(6-(3-ethoxyphenyl)-5-methylpyridin-3-ylamino)-5-methylbenzoic acid;
2-(6-(3-methoxyphenyl)-5-methylpyridin-3-ylamino)-5-methylbenzoic acid;
5-methyl-2-(5-methyl-6-phenylpyridin-3-ylamino)benzoic acid;
2-(6-(3-isopropoxyphenyl)-5-methylpyridin-3-ylamino)-5-methylbenzoic acid;
5-cyclopropyl-2-(5-methyl-6-(3-(trifluoromethoxy)phenyl)pyridin-3-ylamino)benzoic acid;
5-cyclopropyl-2-(5-methyl-6-phenylpyridin-3-ylamino)benzoic acid;
2-(6-(2-fluorophenyl)-5-methylpyridin-3-ylamino)-5-methylbenzoic acid;
5-cyclopropyl-2-(6-phenyl-5-(trifluoromethyl)pyridin-3-ylamino)benzoic acid;
5-cyclopropyl-2-(6-(3-methoxyphenyl)-5-(trifluoromethyl)pyridin-3-ylamino)benzoic acid;
2-(6-(2-chlorophenyl)pyridin-3-ylamino)-5-cyclopropylbenzoic acid;
2-(2-(2-chlorophenyl)pyrimidin-5-ylamino)-5-cyclopropylbenzoic acid;
2-(2-(2-chlorophenyl)pyrimidin-5-ylamino)-5-methylbenzoic acid;
2-(6-(2,6-difluorophenyl)pyridin-3-ylamino)-5-methylbenzoic acid;
5-methyl-2-(5-methyl-6-(3-(pyrrolidine-1-carbonyl)phenyl)pyridin-3-ylamino)benzoic acid;
2-(6-(3-(cyclopropylcarbamoyl)phenyl)-5-methylpyridin-3-ylamino)-5-methylbenzoic acid;
5-cyclopropyl-2-(2-(2-fluorophenyl)pyrimidin-5-ylamino)benzoic acid;
and their pharmaceutically acceptable salts, their solvates, their N-oxides, their stereoisomers or their deuterated derivates thereof.

Even more preferably, when the amine derivative of formula (I) A-NH-B is the azabiphenylaminobenzoic acid derivative of formula (I-b), it is selected from the group consisting of
5-Methyl-2-(6-(3-(trifluoromethyl)phenyl)pyridin-3-ylamino)benzoic acid, 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid, 5-methyl-2-((6-(2,3-difluorophenyl)pyridin-3-yl)amino)benzoic acid, 2-(6-(2,6-Difluorophenyl)pyridin-3-ylamino)-5-methylbenzoic acid, 2-(6-(3-(Cyclopropylcarbamoyl)phenyl)-5-methylpyridin-3-ylamino)-5-methylbenzoic,
and their pharmaceutically acceptable salts, their solvates, their N-oxides, their stereoisomers or their deuterated derivates thereof.

Most preferably, when the amine derivative of formula (I) A-NH-B is the azabiphenylaminobenzoic acid derivative of formula (I-b), it is selected from the group consisting of 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid, 5-methyl-2-((6-(2,3-difluorophenyl)pyridin-3-yl)amino)benzoic acid,
and their pharmaceutically acceptable salts, their solvates, their N-oxides, their stereoisomers or their deuterated derivates thereof.

Preferred pharmaceutically acceptable salts of the azabiphenylaminobenzoic acid derivative of formula (I-b) are formed with the pharmaceutically acceptable bases selected from tromethamine, sodium hydroxide or sodium methoxide, being tromethamine and sodium hydroxide particularly preferred.

Of particular interest are the salts of the azabiphenylaminobenzoic acid derivative of formula (I-b) selected from the group consisting of 5-cyclopropyl-2-(2-(2,6-difluorophenyl)pyrimidin-5-ylamino)benzoic acid, tromethamine salt; 5-cyclopropyl-2-(2-(2,6-difluorophenyl)pyrimidin-5-ylamino)benzoic acid, sodium salt; 5-cyclopropyl-2-((2-(2-(trifluoromethyl)phenyl)pyrimidin-5-yl)amino)benzoic acid tromethamine salt; and 5-methyl-2-((6-(2,3-difluorophenyl)pyridin-3-yl)amino)benzoic acid, tromethamine salt.

In a preferred embodiment, the amine derivative of formula (I) A-NH-B is selected from the group consisting of amino(iso)nicotinic acid derivatives of formula (I-a) selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid, 2-(3,5-difluoro-2-methylbiphenyl-4-ylamino)nicotinic acid; and azabiphenylaminobenzoic acid derivatives of formula (I-b) selected from the group consisting of 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid, 5-methyl-2-((6-(2,3-difluorophenyl)pyridin-3-yl)amino)benzoic acid, and their pharmaceutically acceptable salts, their solvates, their N-oxides, their stereoisomers or their deuterated derivates thereof. Preferably, the amine derivative of formula (I) A-NH-B is selected from the group consisting of amino(iso)nicotinic acid derivatives of formula (I-a) selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid, 2-(3,5-difluoro-2-methylbiphenyl-4-ylamino)nicotinic acid; and azabiphenylaminobenzoic acid derivatives of formula (I-b) selected from the group consisting of 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid, 5-methyl-2-((6-(2,3-difluorophenyl)pyridin-3-yl)amino)benzoic acid, and their pharmaceutically acceptable salts, their solvates, their N-oxides, their stereoisomers or their deuterated derivates thereof.

In a further preferred embodiment, the amine derivative of formula (I) A-NH-B is selected from the group consisting of 2-(3,5-difluoro-3'-methoxybiphenyl-4-ylamino)nicotinic acid and 5-cyclopropyl-2-(2-(2,6-difluorophenyl)pyrimidin-5-ylamino)benzoic acid, and their pharmaceutically acceptable salts, their solvates, their N-oxides, their stereoisomers or their deuterated derivates thereof.

### The skin disease or disorder

The present invention provides an amino derivative of formula (I) A-NH-B for use in the treatment of proliferative skin diseases or disorders.

As used herein, the term proliferative skin disease or disorder refers to a skin disease or disorder characterized by accelerated cell division in the epithelium of the skin (epidermis) and the support tissues of the skin (dermis) or appendages thereto, and which are associated with incomplete tissue differentiation (Vooorhees et al., The Journal of Investigate Dermatology, 1976, 67(3), pp 442-480)

Typically, the skin disease or disorder is other than psoriasis, contact dermatitis and atopic dermatitis.

More typically, the skin disease or disorder is not an inflammatory disease or disorder.

Further, the skin disease or disorder is typically not an autoimmune disease or disorder.

Preferably, the skin disease or disorder is not an autoimmune or inflammatory disease or disorder.

Typically, the disease or disorder is selected from a) epidermal and follicular tumors (such as basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma); b) sebaceous gland tumors (such as sebaceous carcinoma); c) sweat gland tumors (such as mammary Paget's disease, extramammary Paget's disease, eccrine porocarcinoma, microcystic adnexal carcinoma (MAC) or mucinous carcinoma of the skin); d) nervous system tumors (such as Merkel cell carcinoma (MCC)); e) mesenchymal tumors (such as dermatofibrosarcoma protuberans (DFSP), malignant fibrous histiocytoma (MFH), atypical fibroxanthoma, epithelioid sarcoma, synovial sarcoma, liposarcoma, angiosarcoma, Kaposi's sarcoma, cutaneous lymphoma or multiple myeloma); f) metastatic carcinoma of the skin; g) skin melanoma; h) lamellar ichthyosis; i) acne; or j) hirsutism.

Preferably, the skin disease or disorder is selected from the group consisting of basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma.

In a preferred embodiment, the skin disease or disorder is selected from the group consisting of basal cell carcinoma (BCC), squamous cell carcinoma (SCC) and actinic keratosis (AK); preferably the skin disease or disorder is actinic keratosis (AK).

Typically, the amine derivative of formula (I) A-NH-B as defined previously is administered in a suitable form for oral, inhalation, topical, transdermal, nasal, rectal, percutaneous or injectable administration.

Preferably, the amine derivative of formula (I) A-NH-B as defined previously is topically or transdermally administered.

As used herein, topical administration describes a route of introducing drugs into the body in which the drug is applied to the skin (cutaneously) for a local (topical) effect, with little or no systemic absorption of the drug.

As used herein, transdermal administration describes a route of introducing drugs into the body in which the drug is delivered through the skin by a patch, a plaster, a pledglet or a pad (transdermally) for a systemic effect. In this way, the drug can be delivered slowly and continuously for many hours or days or even longer. As a result, levels of a drug in the blood can be kept relatively constant.

In a preferred embodiment, the amine derivative of formula (I) A-NH-B as defined previously is topically administered.

### The topical pharmaceutical composition

The invention further relates to a topical pharmaceutical composition comprising the amine derivative of formula (I) A-NH-B as defined previously in association with a pharmaceutically acceptable vehicle or carrier.

Examples of suitable vehicles or carriers according to the invention include water and oils. According to the invention, an oil is a substance that is in a viscous liquid state ("oily") at room temperature or slightly warmer, and is both hydrophobic (immiscible with water) and lipophilic (miscible with other oils). Suitable oil phases according to the invention are petroleum hydrocarbons (mineral oils, paraffins and waxes), animal and vegetable fats and oils, fatty acids, fatty alcohols, natural waxes, silicones and polyols other than hexylene glycol, or mixtures thereof.

Suitable petroleum hydrocarbons, i.e. mineral oils, paraffins and waxes from petroleum according to the present invention are: hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffines waxes (which consist of C₄₀-C₅₅ compounds which contain, in addition to normal hydrocarbons, large amounts of iso-alkanes and naphtenes with long alkyl side-chains, the iso-alkanes forming microcrystals, the microcrystalline paraffines waxes having mean molecular weights comprised between 500 and 800 g/mol, being solids at room temperature, and having melting points comprised between 60°C and 90°C), or mixtures thereof. Preferred petroleum hydrocarbons are hard paraffin, liquid paraffin, light liquid paraffin, white soft paraffin or mixture thereof, being particularly preferred liquid paraffin, white soft paraffin or mixtures thereof.

Suitable animal or vegetable fats and oils according to the present invention are esters of linear and/or branched, saturated and/or unsaturated alkanecarboxylic acids with a chain length of 1 up to 30 carbon atoms and linear and/or branched, saturated and/or unsaturated alcohols with a chain length of 1 up to 30 carbon atoms; or are esters of aromatic carboxylic acids and linear and/or branched, saturated and/or unsaturated alcohols with a chain length of 1 up to 30 carbon atoms.

These oils can be advantageously selected from the group consisting of isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2- ethylhexyl isostearate, 2-octyldodecyl palmitate, cetyl palmitate, oleyl oleate, oleyl erucate, erucyl oleate, erucyl erucate, as well as synthetic, semisynthetic and natural mixtures of such esters, such as jojoba oil (a natural mixture of esters of monounsaturated monocarboxylic acids with a C₁₈-C₂₄ chain with also monounsaturated monoalcohols and with a long C₁₈-C₂₄ chain).

Other suitable oils of the type of esters of saturated alkanecarboxylic acids and alcohols are fatty acid methyl esters, preferably C₆-C₂₄ fatty acid methyl esters from animal and vegetable fats and oils such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty acids such as caproic acid, caprylic acid, capric acid, lauric acid, myristic acid, palmitic acid (cetylic acid), palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable animal or vegetable fats and oils according to the present invention are fatty acid triglycerides, specifically triglycerin esters of linear and/or branched, saturated and/or unsaturated alkanecarboxylic acids with a chain length of 6 up to 24 carbon atoms, preferably of 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The triglycerides more simple are those constituted by a sole fatty acid.

Fatty acid triglycerides can be advantageously chosen, for example, from the group consisting of synthetic, semi-synthetic and natural oils, as for example, animal fats and oils such as cow tallow, pig lard, bone oil, aquatic animal fats and oils (fish, such as herring, cod or sardine; cetaceans; etc.); and vegetable fats and oils such as avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its by-products such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

Particularly preferred are vegetable fats and oils as described above.

Suitable fatty acids according to the present invention are C₆-C₂₄ fatty acids from vegetable and animal fats and oils, such as those previously described, such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty acids such as caproic acid, caprylic acid, capric acid, lauric acid, myristic acid, palmitic (cetylic) acid, palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or or technical grade mixtures thereof. Fatty acids of the lauric, myristic, palmitic, palmitoleic, stearic, isostearic, 2-ethylhexanoic, oleic, ricinoleic, behenic type, or mixtures thereof are preferred, in particular, those from vegetable origin.

Suitable fatty alcohols according to the present invention are C₆-C₂₄ fatty alcohols from vegetable and animal fats and oils such as those previously described, such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty alcohols such as caproyl alcohol, capryl alcohol, capric alcohol, lauryl alcohol, myristyl alcohol, palmytil (cetyl) alcohol, palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures thereof such as cetostearyl alcohol. Fatty alcohols of the lauryl, myristyl, palmityl, palmitoleyl, stearyl, isostearyl 2-ethylhexanoyl, oleyl, ricinoleyl and behenyl type, or technical grade mixtures thereof such as cetostearyl alcohol are preferred, in particular, those from vegetable origin.

Suitable natural waxes according to the present invention are the candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

Silicones suitable according to the present invention are cyclic and/or linear silicones, which can be found as monomers generally characterized by structural elements such as: 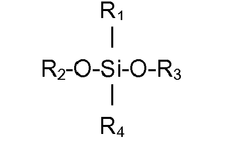 where the silicon atoms can be substituted by alkyl or aryl radicals equal or different, represented here generally by R₁-R₄ groups.

Linear silicones with siloxane units suitable according to the present invention are generally characterized by structural elements such as: 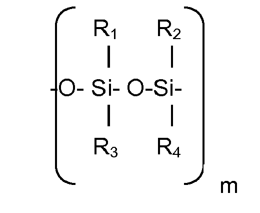 where the silicon atoms can be substituted by alkyl or aryl radicals equal or different, are represented here in general by R₁-R₄ groups (meaning the number of different radicals is not necessarily limited to 4), m can take values from 2 to 200.000.

Cyclic silicones suitable according to the present invention are generally characterized by structural elements such as: 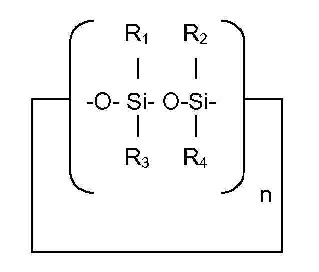 where the silicon atoms can be substituted by alkyl or aryl radicals equal or different, represented here generally by R₁-R₄ groups (meaning the number of different radicals is not necessarily limited to 4), n can take values of 3/2 to 20. Fractional values of n indicate that it may be odd numbers of siloxane groups present in the ring.

Specific examples include a cyclic methyl siloxane having the formula [(CH₃)₂SiO]ₓ in which x is 3-6, or short chain linear methyl siloxanes having the formula ((CH₃)₂SiO[(CH₃)₂SiO]ySi(CH₃)₃ in which y is 0-5.

Some suitable cyclic methyl siloxanes are hexamethylcyclotrisiloxanes (D3), a solid with a boiling point of 134°C and the formula [(Me₂)SiO]₃; octamethylcyclotetrasiloxane (D4) with a boiling point of 176°C, a viscosity of 2.3 mm²/s, and the formula [(Me₂)SiO]₄; decamethylcyclopentasiloxane (D5) (cyclomethicone) with a boiling point of 210°C, a viscosity of 3.87 mm²/s, and the formula [(Me₂)SiO]₅; and dodecamethylcyclohexasiloxane (D6) with a boiling point of 245°C, a viscosity of 6.62 mm²/s and the formula [(Me₂)SiO]₆.

Some suitable short linear methyl siloxane are hexamethyldisiloxane (MM) with a boiling point of 100°C, viscosity of 0-65 mm²/s, and formula Me₃SiOMe₃; octamethyltrisiloxane (MDM) with a boiling point of 152°C, viscosity of 1.04 mm²/s, and formula Me₃SiOMe₂SiOSiMe₃; decamethyltetrasiloxane (MD2M) with a boiling point of 194°C, viscosity of 1.53 mm²/s, and formula Me₃SiO(MeSiO)₂SiMe₃; dodecamethylpentasiloxane (MD3M) with a boiling point of 229°C, viscosity of 2.06 mm²/s, and formula Me₃SiO(Me₂SiO)₃SiMe₃; tetradecamethylhexasiloxane (MD4M) with a boiling point of 245°C, viscosity of 2.63 mm²/s, and formula Me₃SiO(Me₂SiO)₄SiMe₃; and hexadecamethylheptasiloxane (MD5M) with a boiling point of 270°C, viscosity of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, dimethiconol, cyclomethicone (octametilciclotetrasiloxane), hexamethylcyclotrisiloxane, poly(dimethylsiloxane), cetyldimethicone and behenoxy dimethicone are also included.

In addition, mixtures of cyclomethicone and isotridecyl isononanoate and of cyclomethicone and 2-ethylhexyl isostearate are also suitable silicones according to the invention.

Suitable polyols other than hexylene glycol according to the present invention are preferably water-soluble polyols such as polyhydric alcohols with two or more hydroxyl groups in their molecule. Specific examples can include ethylene glycol, propylene glycol, 1,3- butylene glycol, 1,4-butylene glycol, dipropylene glycol, polyethylene glycol with average molecular weights by weight ranging between 100 and 1000, glucose, fructose, galactose, mannose, ribose, erythrose, maltose, maltitose, maltotriose, sucrose, xylitol, sorbitol, threitol, erythritol, glycerol, polyglycerol and starch alcohols. Preferred polyols other than hexylene glycol are ethylene glycol, propylene glycol, 1,3-butylene glycol, 1,4-butylene glycol, dipropylene glycol, polyethylene glycol with average molecular weights by weight ranging between 100 and 1000, glycerol, polyglycerol, and mixtures thereof.

The pH value of the topical pharmaceutical composition according to the invention is typically within the acceptable range for topical administration, and is preferably in the range of 3.0 to 6.0, more preferably in the range of 3.5 to 5.0.

In a preferred embodiment, the topical pharmaceutical composition according to the invention is formulated in the form of a cream, a gel, an ointment, a paste, a suspension, a lotion, a foam, a spray, an aerosol or a solution.

The viscosity of the topical pharmaceutical composition according to the invention will depend on the form of the composition. For instance, in the case of a cream, the viscosity is typically in the range of 2,000 to 15,000 mPa.s, preferably in the range of 2,500 to 10,000 mPa.s, more preferably in the range of 3,000 to 7,000 mPa.s measured at 20°C using a DIN-Rotations Rheometer (Paar Physica); Measuring System Z 3 DIN ; D= 57 1/s.

In the case of a gel, the viscosity is typically in the range of 300 to 1,500 mPa.s, preferably in the range of 500 to 1,200 mPa.s, more preferably in the range of 600 to 900 mPa.s measured at 20°C using a DIN-Rotations Rheometer (Paar Physica); Measuring System Z 3 DIN ; D= 57.2/s.

The topical pharmaceutical compositions according to the invention may optionally further comprise one or more active ingredients selected from the group consisting of (a) antibiotics, (b) antifungal agents, (c) antihistamine agents, (d) antimetabolites, (e) corticosteroids, (f) immunosuppressants, (g) nonsteroidal anti-inflammatory drugs (NSAIDs), (h) vitamin A analogues, and (i) vitamin D analogues.

Examples of suitable antibiotics for use in the context of the present invention include, but are not limited to aclarubicin, actinomycin D, amrubicin, annamycin, adhamycin, bleomycin, daunorubicin, doxorubicin, elsamitrucin, epirubicin, galarubicin, idarubicin, mitomycin C, mupiricin, nemorubicin, neocarzinostatin, peplomycin, pirarubicin, rebeccamycin, retapamulin, stimalamer, streptozocin, valrubicin, zinostatin and combinations thereof.

Examples of suitable antifungal agents for use in the context of the present invention include, but are not limited to amphotericin B, bifonazole, caspofungin, clotrimazole, echinocandin B, econazole, fluconazole, flucytosine, itraconazole, ketoconazole, miconazole, posaconazole, ravuconazole, terbinafine, tioconazole, voriconazole and combinations thereof.

Examples of suitable antihistamine agents for use in the context of the present invention include, but are not limited to clemastine, diphenhydramine (benadryl), doxylamine, loratadine, desloratadine, fexofenadine, pheniramine, cetirizine, ebastine, promethazine, chlorpheniramine, levocetirizine , olopatadine, quetiapine, meclizine, dimenhydrinate, embramine, dimethindene, dexchlorpheniramine, cimetidine, famotidine, ranitidine, nizatidine, roxatidine, lafutidine, A-349,821, ABT-239, ciproxifan, clobenpropit, thioperamide, JNJ 7777120, VUF-6002 and combinations thereof.

Examples of suitable antimetabolites for use in the context of the present invention include, but are not limited to methotrexate, 6-mercaptopuhne riboside, mercaptopurine, 5-fluorouracil (5-FU) alone or in combination with leucovorin, tegafur, UFT, doxifluhdine, carmofur, cytarabine, cytarabine ocfosfate, enocitabine, S-1, Alimta (premetrexed disodium, LY231514, MTA), Gemzar (gemcitabine, Eli Lilly), fludarabin, 5-azacitidine, capecitabine, cladribine, clofarabine, decitabine, eflornithine, ethynylcytidine, cytosine arabinoside, hydroxyurea, TS-1 , melphalan, nelarabine, nolatrexed, ocfosfate, disodium premetrexed, pentostatin, pelitrexol, raltitrexed, thapine, trimetrexate, vidarabine, vincristine, vinorelbine, N-(5-[N-(3,4-dihydro-2-methyl-4-oxoquinazolin-6- ylmethyl)-N-methylamino]-2-thenoyl)-L-glutamic acid, and combinations thereof. Preferred antimetabolites are methotrexate and 5-fluorouracil (5-FU), being 5-fluorouracil (5-FU) particularly preferred.

Examples of suitable corticosteroids and glucocorticoids for use in the context of the present invention include, but are not limited to prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, ioteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11 beta-hydroxy-17alpha-[2-(methylsulfanyl)acetoxy]-4-pregnene-3,20-dione, desisobutyrylciclesonide, hydrocortisone acetate, hydrocortisone sodium succinate, prednisolone sodium phosphate and hydrocortisone probutate, prednisolone sodium metasulfobenzoate, clobetasol propionate, and combinations thereof. Preferred corticosteroids and glucocorticoids are prednisolone, prednicarbate, mometasone furoate and derivatives, esters, salts and mixtures thereof, and combinations thereof.

Examples for suitable immunosupressants include, but are not limited to imiquimod, picremolimus, tacrolimus, cyclosporine A, anti-TNF agents, and combinations thereof. A preferred immunosupressant is imiquimod.

Examples of suitable nonsteroidal anti-inflammatory drugs (NSAIDs) for use in the context of the present invention include, but are not limited to oxicams, piroxicam, meloxicam, isoxicam, tenoxicam, sudoxicam, CP-14,304, salicylates, aspirin (acetylsalicylic acid), disalcid, benorylate, trilisate, safapryn, solprin, diflunisal, fendosal, acetic acid derivatives, aceclofenac, diclofenac, fenclofenac, indomethacin, sulindac, tolmetin, isoxepac, furofenac, tiopinac, zidometacin, acematacin, fentiazac, zomepirac, clindanac, oxepinac, felbinac, etodolac, ketorolac, fenamates, mefenamic, meclofenamic, flufenamic, niflumic, tolfenamic acids, propionic acid derivatives, ibuprofen, naproxen, benoxaprofen, flurbiprofen, ketoprofen, piketoprofen, fenoprofen, fenbufen, indopropfen, pirprofen, carprofen, oxaprozin, pranoprofen, miroprofen, tioxaprofen, suprofen, alminoprofen, tiaprofen, pyrazoles, phenylbutazone, oxyphenbutazone, feprazone, azapropazone, trimethazone and derivatives, esters, salts and mixtures thereof, and combinations thereof. Preferred NSAIDs are aspirin (acetylsalicylic acid), aceclofenac, diclofenac, and derivatives, esters, salts and mixtures thereof, and combinations thereof. A preferred NSAID is diclofenac sodium.

Examples of suitable vitamin A analogues for use in the context of the present invention include, but are not limited to acitretin, isotretinoin, tretinoin, isotretinoin, fenretinide and derivatives, esters, salts and mixtures thereof, and a combination thereof. A preferred vitamin A analogue is tretinoin.

Examples of suitable vitamin D analogues for use in the context of the present invention include, but are not limited to alfacalcidol, calcipotriol, calcitriol, dihydrotachysterol-2, doxercalciferol, holecalciferol, 22-oxacalcitriol, paracalcitol, seocalcitol (EB 1089), tacalcitrol, and combinations thereof.

In an embodiment, the topical pharmaceutical compositions according to the invention comprise i) an amine derivative of formula (I) A-NH-B selected from the group consisting of 2-(3,5-difluoro-3'-methoxybiphenyl-4-ylamino)nicotinic acid and 5-cyclopropyl-2-(2-(2,6-difluorophenyl)pyrimidin-5-ylamino)benzoic acid, and their pharmaceutically acceptable salts, their solvates, their N-oxides, their stereoisomers or their deuterated derivates thereof; and ii) 5-fluorouracil (5-FU).

In another embodiment, the topical pharmaceutical compositions according to the invention comprise i) an amine derivative of formula (I) A-NH-B selected from the group consisting of 2-(3,5-difluoro-3'-methoxybiphenyl-4-ylamino)nicotinic acid and 5-cyclopropyl-2-(2-(2,6-difluorophenyl)pyrimidin-5-ylamino)benzoic acid, and their pharmaceutically acceptable salts, their solvates, their N-oxides, their stereoisomers or their deuterated derivates thereof; and ii) imiquimod.

In another embodiment, the topical pharmaceutical compositions according to the invention comprise i) an amine derivative of formula (I) A-NH-B selected from the group consisting of 2-(3,5-difluoro-3'-methoxybiphenyl-4-ylamino)nicotinic acid and 5-cyclopropyl-2-(2-(2,6-difluorophenyl)pyrimidin-5-ylamino)benzoic acid, and their pharmaceutically acceptable salts, their solvates, their N-oxides, their stereoisomers or their deuterated derivates thereof; and ii) aspirin (acetylsalicylic acid).

In another embodiment, the topical pharmaceutical compositions according to the invention comprise i) an amine derivative of formula (I) A-NH-B selected from the group consisting of 2-(3,5-difluoro-3'-methoxybiphenyl-4-ylamino)nicotinic acid and 5-cyclopropyl-2-(2-(2,6-difluorophenyl)pyrimidin-5-ylamino)benzoic acid, and their pharmaceutically acceptable salts, their solvates, their N-oxides, their stereoisomers or their deuterated derivates thereof; and ii) diclofenac sodium.

In another embodiment, the topical pharmaceutical compositions according to the invention comprise i) an amine derivative of formula (I) A-NH-B selected from the group consisting of 2-(3,5-difluoro-3'-methoxybiphenyl-4-ylamino)nicotinic acid and 5-cyclopropyl-2-(2-(2,6-difluorophenyl)pyrimidin-5-ylamino)benzoic acid, and their pharmaceutically acceptable salts, their solvates, their N-oxides, their stereoisomers or their deuterated derivates thereof; and ii) tretinoin.

The topical pharmaceutical compositions according to the invention may optionally further comprise other well-known pharmaceutically and/or cosmetically acceptable additives, such as, e.g. anti-irritants, antioxidants, buffering agents (pH adjusting agents), chelating agents, emollients, penetration enhancing agents, preservative agents, solubilizing agents, thickening agents, wetting agents, and the like, or mixtures thereof.

Examples of suitable anti-irritants are aloe vera, chamomile, alpha-bisabolol, cola nitida extract, green tea extract, tea tree oil, licoric extract, batyl alcohol (α-octadecyl glyceryl ether), selachyl alcohol (α-9-octadecenyl glyceryl ether), chimyl alcohol (α-hexadecyl glyceryl ether), panthenol, allantoin, caffeine or other xanthines, glycyrrhizic acid and derivatives thereof, and mixtures thereof.

Antioxidants used can be any antioxidants which are suitable or customary for cosmetic and/or dermatological applications. Suitable antioxidants are advantageously selected from the group consisting of amino acids (for example glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. α-carotene, β-carotene, lycopene) and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts) and sulphoximine compounds (e.g. buthionine sulphoximines, homocysteine sulphoximine, buthionine sulphones, penta-, hexa-, heptathionine sulphoximine) in very small tolerated doses (e.g. pmol to µmol/kg), also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), and coniferylbenzoate of benzoin, rutinic acid and derivatives thereof, ferulic acid and derivatives thereof, butylated hydroxytoluene, butylated hydroxyanisole, nordihydroguaiac resin acid, nordihydroguaiaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenium methionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

Any pharmaceutically acceptable buffering agents to adjust the pH of the aqueous liquid pharmaceutical compositions according to the invention to be within the acceptable range for topical administration, preferably in the range of 3.0 to 6.0, more preferably in the range of 3.5 to 5, can be used. For example the inclusion in the composition of a pharmaceutically acceptable acid such as acetic, citric, fumaric, phosphoric, hydrochloric, lactic or nitric acids or the like, or a mixture thereof. It will also be understood that certain compositions of the invention can have a pH in the desired range without inclusion of a pH adjusting agent specifically for that purpose. Typically, however, an acidic buffer system is present in the composition to achieve the desired pH. An acidic buffer system comprises an acidulant and a buffering agent. Suitable acidulants will be known to those of skill in the art and illustratively include acetic, citric, fumaric, hydrochloric, phosphoric, lactic and nitric acids and the like, and mixtures thereof. Suitable buffering agents will likewise be known to those of skill in the art and illustratively include potassium metaphosphate, potassium phosphate, sodium phosphate, sodium acetate, sodium citrate and the like, and mixtures thereof.

Suitable emollients, which can be used in the composition of the present invention include, for example, dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil, polyol carboxylic acid esters, derivatives thereof, and the like, and combinations thereof.

Examples of suitable penetration enhancing agents can include, e.g., dimethylsulfoxide (DMSO), N-methyl pyrrolidine, dimethyl formamide (DMF), allantoin, urazole, N,N-dimethylacetamide (DMA), decylmethylsulfoxide, polyethylene glycol monolaurate, propylene glycol, propylene glycol monolaurate, glycerol monolaurate, lecithin, the 1-substituted azacycloheptan-2-ones, particularly 1-n-dodecylcyclazacycloheptan-2-one, alcohols, glycerin, hyaluronic acid, transcutol, and the like, and combinations thereof. Certain oil components (e.g., certain vegetable oils such as, e.g., safflower oil, cottonseed oil and corn oil) also can exhibit penetration enhancing properties.

Examples of suitable preservatives to prevent microbial contamination are alkylparabens, particularly methylparaben, propylparaben and butylparaben; sodium benzoate; butylated hydroxy toluene; butylated hydroxyanisole; ethylenediamine tetraacetic acid; chlorobutanol; benzyl alcohol; phenylethylalcohol; dehydroacetic acid; sorbic acid; potassium sorbate; benzalkonium chloride; benzethonium chloride; and mixtures thereof. The amount of preservative generally utilized will vary depending upon the preservative selected.

Examples of solubilizing agents are, for example, nonionic surfactants from at least one of the following groups: products of the addition of 1 to 30 moles of ethylene oxide and/or 0 to 5 moles of propylene oxide onto linear C₈-C₂₂ fatty alcohols, C₁₂-C₂₂ fatty acids and alkyl phenols containing 8 to 15 carbons in the alkyl group; alkyl and/or alkenyl oligoglycosides containing 8 to 22 carbons in the alkyl group and ethoxylated analogs thereof; addition products of 1 to 15 moles of ethylene oxide with castor oil and/or hydrogenated castor oil; addition products of 15 to 60 moles of ethylene oxide with castor oil and/or hydrogenated castor oil; partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide; mixtures of alkoxylated glycerides and alkoxylated glycerine, partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3 to 18 carbons and addition products thereof with 1 to 30 moles of ethylene oxide; mixed esters of pentaerythritol, fatty acids, citric acid and fatty alcohol and/or mixed esters of fatty acids containing 6 to 22 carbons, methyl glucose and polyols, preferably glycerol or polyglycerol; mono-, di- and trialkyl phosphates and mono-, di- and/or tri-PEG-alkyl phosphates and salts thereof; block copolymers, for example Polyethyleneglycol-30 Dipolyhydroxystearate; polymer emulsifiers; polyalkylene glycols and alkyl glyceryl ethers. Particularly preferred solubilizing agents are products of the addition of 1 to 30 moles of ethylene oxide and/or 0 to 5 moles of propylene oxide onto linear C₈-C₂₂ fatty alcohols such as lauryl, myristyl, cetyl (palmityl), stearyl, oleyl, and ricinoleyl alcohols, or technical grade mixtures thereof such as cetostearyl alcohol or palmitoleyl alcohol.

A thickening agent or viscosity-enhancing agent can be included to generally thicken the liquid pharmaceutical compositions. While any suitable thickening agent can be included in the compositions of the present invention, a preferred thickening agent, when used, includes one or more of acacia, alginic acid bentonite, carbomer, carboxymethylcellulose calcium or sodium, cetostearyl alcohol, methyl cellulose, ethylcellulose, glycerin, gelatin guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium starch glycolate, starch tragacanth, and xanthan gum, and any combination thereof. More preferred thickening agents are glycerin, hydroxypropylmethylcellulose, and xanthan gum, and any combination thereof.

Examples of wetting agents (chemical substances that increase the spreading and penetrating properties of a liquid by lowering its surface tension) include one or more cationic surfactants, such as benzalkonium chloride; non-ionic surfactants such as polyoxyethylene and polyoxypropylene block copolymers; polyoxyethylene fatty acid glycerides and oils (such as polyoxyethylene (6) caprylic/capric mono- and diglycerides), polyoxyethylene (40) hydrogenated castor oil; polyoxyethylene sorbitan esters, such as polysorbate 20 and polysorbate 80; propylene glycol fatty acid esters, such as propylene glycol laureate; glyceryl fatty acid esters, such as glyceryl monostearate; sorbitan esters, such as sorbitan monolaurate, sorbitan monooleate, sorbitan monopalmitate and sorbitan monostearate; glyceryl fatty acid esters, for example glyceryl monostearate; anionic surfactants such as sodium lauryl sulphate, sodium lauryl ether sulphate; or fatty acids and salts thereof, such as oleic acid, sodium oleate and triethanolamine oleate.

In the topical pharmaceutical compositions according to the invention the amount of the amine derivative of formula (I) A-NH-B as defined previously, is typically in the range of 0.01 to 15 wt.%, preferably in the range of 0.02 to 10 wt.%, more preferably in the range of 0.05 to 7.5 wt.%, even more preferably in the range of 0.1 to 6 wt.% based on the total weight of the composition.

The present invention provides an amine derivative of formula (I) A-NH-B for use in the treatment of proliferative skin diseases or disorders, in particular wherein the disease or disorder is selected from a) epidermal and follicular tumors (such as basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma); b) sebaceous gland tumors (such as sebaceous carcinoma); c) sweat gland tumors (such as mammary Paget's disease, extramammary Paget's disease, eccrine porocarcinoma, microcystic adnexal carcinoma (MAC) or mucinous carcinoma of the skin); d) nervous system tumors (such as Merkel cell carcinoma (MCC)); e) mesenchymal tumors (such as dermatofibrosarcoma protuberans (DFSP), malignant fibrous histiocytoma (MFH), atypical fibroxanthoma, epithelioid sarcoma, synovial sarcoma, liposarcoma, angiosarcoma, Kaposi's sarcoma, cutaneous lymphoma or multiple myeloma); f) metastatic carcinoma of the skin; g) skin melanoma; h) lamellar ichthyosis; i) acne; or j) hirsutism.

The invention further provides a topical pharmaceutical composition comprising an amine derivative of formula (I) A-NH-B alone or in combination with one or more active ingredients for use in the treatment of proliferative skin diseases or disorders, in particular wherein the disease or disorder is as defined above.

The invention also provides a method for treating a subject afflicted with a proliferative skin disease or disorder, which comprises applying to the affected area of the skin of said subject an effective amount of an amine derivative of formula (I) A-NH-B, or a pharmaceutical composition comprising an amine derivative of formula (I) A-NH-B alone or in combination with one or more active ingredients.

Typically, the affected area of the skin affected by the disease or disorder is selected from the group consisting of the face, ears, scalp, neck, forearms, back, legs, arms and hands.

The method of using the topical pharmaceutical composition of the invention is by applying it to completely cover the affected area, forming an occlusive barrier. The amount needed depends on the size of the lesion.

The usual frequency of application ranges between 1 and 4 times a day, for a period that ranges between 10 and 120 days.

The invention further relates to the use of an amine derivative of formula (I) A-NH-B for the manufacture of a medicament for the treatment of a proliferative skin disease or disorder, in particular wherein the proliferative disease or disorder is as defined above.

The following examples are given in order to provide a person skilled in the art with a sufficiently clear and complete explanation of the present invention, but should not be considered as limiting of the essential aspects of its subject, as set out in the preceding portions of this description.

### EXAMPLES

### Example 1

### Proliferation assay

Human adult low calcium high temperature (HaCaT) cells are spontaneously transformed human keratinocytes that have the characteristics of basal epidermal keratinocytes (Boukamp et at. J Cell Biol 106:761-771, 1988) This line can be used as *in vitro* model for highly proliferative epidermis (Ockenfels et at., Arch Dermatol Res 287:301-309, 1995)

HaCaT cells exponentially growing were plated in clear bottom, white walled, 96-well plates at a density of 5000 cells/well in 100 µL Dulbecco's modified Eagle's medium (DMEM) containing 1% fetal bovine serum (FBS), and incubated overnight at 37 °C, 5% CO2. Next day, media in each well was replaced with 50 µL growth medium containing 10% FBS. 50 µL of compounds at two-fold the desired concentration in growth medium containing 2% dimethyl sulfoxide (DMSO) were added to the wells. Six wells were replaced with fresh 100 µL of medium containing 1% FBS and 1% DMSO and were used as a control of no proliferation. Six other wells were replaced with fresh 100 µL of medium containing 10% FBS and 1% DMSO and were used as a control of top proliferation. Each compound concentration was tested in triplicate and ranged from 100 µM to 0.4 µM in half-log serial dilutions. Plates were incubated at 37°C 5% CO₂ for 6 consecutive days. On the day of the assay, media was removed and replaced with 100 µL of phosphate buffered saline (PBS). A bioluminescent assay was performed to assess the intracellular adenosine-5'-triphosphate (ATP) content following the manufacturer instructions (ATP-lite, Perkin Elmer, Belgium). Results were expressed as a % of inhibition of proliferation in relation to the top and bottom proliferation wells. Absolute IC₅₀ corresponded to the concentration of compound that caused 50% inhibition of proliferation. Table 1 depicts the mean and the standard deviation of 3 experiments for each indicated compound.

**Table 1 shows the IC₅₀ of some compounds of the invention and a Comparative Example**

| Reference | Compound | mean IC₅₀ (µM) | standard deviation (µM) |
|---|---|---|---|
| Comparative Example | Teriflunomide | 15.2 | 3.4 |
| Amino derivatives of formula (I) A-NH-B | 2-(3,5-difluoro-3'-methoxybiphenyl-4-ylamino)nicotinic acid (compound of formula (I-a)) | 0.58 | 0.07 |
| | 5-cyclopropyl-2-(2-(2,6-difluorophenyl)pyrimidin-5-ylamino)benzoic acid (compound of formula (I-b)) | 0.26 | 0.03 |

As it can be clearly seen from Table 1, amino derivatives of the invention are more active in inhibiting the proliferation of HaCaT cells (premalignant keratinocytes) than teriflunomide (DHDOH inhibitor which was reported to inhibit HaCaT cells). Thus, it can be concluded that amino derivatives of formula (I) A-NH-B, which are also DHODH inhibitors, are useful in the treatment of proliferative skin diseases or disorders, in particular wherein the disease or disorder is as defined above.

Modifications, which do not affect, alter, change or modify the essential aspects of the compounds, combinations or pharmaceutical compositions described, are included within the scope of the present invention.

## Claims

1. An amino derivative of formula (I), or a pharmaceutically acceptable salt or N-oxide or solvate or deuterated derivative thereof, for use in treating a proliferative skin disease or disorder,
A-NH-B (formula (I))
wherein
A represents a group of formula A1 or A2, 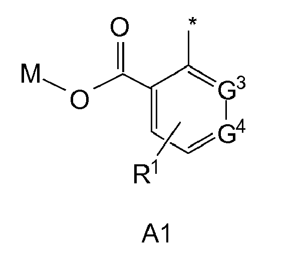 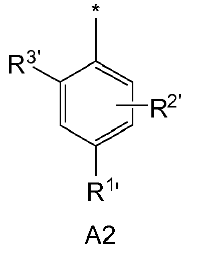 wherein the asterisk symbol indicates the bond that is linked to the -NH-B moiety; and wherein:
- in the group of formula A1:
• R¹ represents a group selected from hydrogen atoms, halogen atoms, C₁₋₄ alkyl groups which may be optionally substituted by 1, 2 or 3 substituents selected from halogen atoms and hydroxy groups, and C₃₋₈ cycloalkyl groups which may be optionally substituted by 1, 2 or 3 substituents selected from halogen atoms and hydroxy groups;
• one of the groups G³ and G⁴ is a nitrogen atom and the other is a CH group;
• M is a hydrogen atom or a pharmaceutically acceptable cation;
- in the group of formula A2:
• R¹, is selected from the group consisting of hydrogen atoms, halogen atoms, C₁₋₄ alkyl, C₃₋₄ cycloalkyl, -CF₃ and -OCF₃;
• R²_{,} is selected from the group consisting of hydrogen atoms, halogen atoms and C₁₋₄ alkyl group;
• R³, is selected from the group consisting of -COOR⁵', -CONHR⁵', tetrazolyl, -SO₂NHR⁵' and -CONHSO₂R⁵' groups, wherein R⁵' is selected from the group consisting of a hydrogen atom and linear or branched C₁₋₄ alkyl groups;
B represents a group of formula B1 or B2, 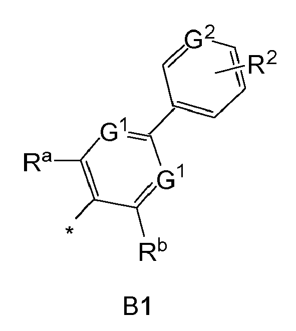 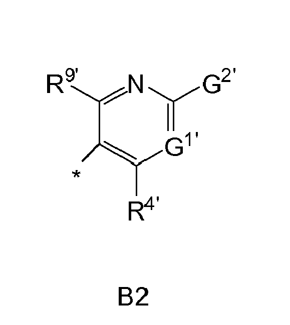 wherein the asterisk symbol indicates the bond that is linked to the A-NH-moiety; and wherein:
- in the group of formula B1
• one of the groups G¹ represents a nitrogen atom or a group CR^{c} and the other represents a group CR^{c};
• G² represents a nitrogen atom or a group CR^{d};
• R² represents a group selected from hydrogen atoms, halogen atoms, hydroxyl groups, C₁₋₄ alkyl groups which may be optionally substituted by 1, 2 or 3 substituents selected from halogen atoms and hydroxy groups, C₁₋₄ alkoxy groups which may be optionally substituted by 1, 2 or 3 substituents selected from halogen atoms and hydroxy groups, and C₃₋8 cycloalkyl groups which may be optionally substituted by 1, 2 or 3 substituents selected from halogen atoms and hydroxy groups;
• R^{a}, R^{b} and R^{c} independently represent groups selected from hydrogen atoms, halogen atoms, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups;
• R^{d} represents a group selected from hydrogen atoms, halogen atoms, hydroxyl groups, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups, and C₃₋₈ cycloalkoxy groups which may be optionally substituted by 1, 2 or 3 substituents selected from halogen atoms and hydroxy groups;
with the proviso that, when at least one of the groups R^{a} and R^{b} represent a hydrogen atom and G² is a group CR^{d}, then R^{d} represents a group selected from C₁₋₄ alkoxy groups which may be optionally substituted by 1, 2 or 3 substituents selected from halogen atoms and hydroxy groups, C₃₋₈ cycloalkoxy groups which may be optionally substituted by 1, 2 or 3 substituents selected from halogen atoms and hydroxy groups;
- in the group of formula B2
• R⁴' is selected from the group consisting of a hydrogen atom, and a C₁₋₄ alkyl group;
• R⁹' is selected from the group consisting of a hydrogen atom and a phenyl group;
• G¹_{,} represents a group selected from N and CR⁶' wherein R⁶' is selected from the group consisting of hydrogen atoms, halogen atoms, C₁₋₄ alkyl, C³⁻⁴ cycloalkyl, C₁₋₄ alkoxy, -CF₃, -OCF₃, monocyclic N-containing C₅₋₇ heteroaryl, monocyclic N-containing C₃₋₇ heterocyclyl groups and a C₆₋₁₀ aryl group which is optionally substituted with one or more substituents selected from halogen atoms and a C₁₋₄ alkyl group;
• G²' represents a group selected from:
• a hydrogen atom, a hydroxy group, a halogen atom, a C₃₋₄ cycloalkyl group, a C₁₋₄ alkoxy group and -NR^{a}'R^{b}', wherein R^{a}' represents a C₁₋₄ alkyl group and R^{b}' is selected from a group consisting of C₁₋₄ alkyl group and C₁₋₄ alkoxy-C₁₋₄ alkyl group, or R^{a}' and R^{b}' together with the nitrogen atom to which they are attached form a saturated 6 to 8 membered heterocyclic ring optionally containing one oxygen atom as an additional heteroatom;
• a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷'R⁸', wherein R⁷' and R³' are independently selected from hydrogen atom, linear or branched C₁₋₄ alkyl group, C₃₋₇ cycloalkyl group, or R⁷' and R⁸' together with the nitrogen atom to which they are attached form a group of formula 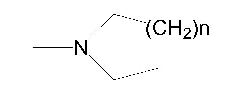 wherein n is an integer from 0 to 3;
and
• a phenyl group which is optionnally substituted by one or more substituents selected from halogen atoms, C₁₋₄ alkyl, hydroxy, C₁₋₄ alkoxy, C₃₋₄ cycloalkyl, C₃₋₄ cycloalkoxy, cyano, -CF₃, -OCF₃, -CONR₇'R₈', oxadiazolyl, triazolyl, pyrazolyl and imidazolyl groups, wherein the oxadiazolyl, triazolyl, pyrazolyl and imidazolyl groups are optionally substituted by C₁₋₄ alkyl or C₃₋₇ cycloalkyl group and wherein R₇' and R₈' are independently selected from hydrogen atom, linear or branched C1-4 alkyl group, a C₃₋₇ cycloalkyl group, or R₇' and R₈' together with the nitrogen atom to which they are attached form a group of formula 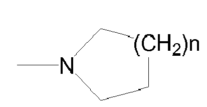 wherein n is an integer from 0 to 3;
• or, when G¹', represents CR⁶', G²' together with R⁶' form a non-aromatic C₅₋₁₀ carbocyclic group or a C₆₋₁₀ aryl group.

2. An amino derivative according to claim 1 selected from the group consisting of amino(iso)nicotinic acid derivatives of formula (I-a) 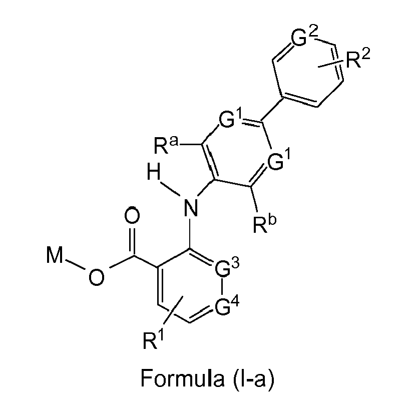 wherein
• one of the groups G¹ represents a nitrogen atom or a group CR^{c} and the other represents a group CR^{c};
• G² represents a nitrogen atom or a group CR^{d};
• R¹ represents a group selected from hydrogen atoms, halogen atoms, C₁₋₄ alkyl groups which may be optionally substituted by 1, 2 or 3 substituents selected from halogen atoms and hydroxy groups, and C₃₋₈ cycloalkyl groups which may be optionally substituted by 1, 2 or 3 substituents selected from halogen atoms and hydroxy groups;
• R² represents a group selected from hydrogen atoms, halogen atoms, hydroxyl groups, C₁₋₄ alkyl groups which may be optionally substituted by 1, 2 or 3 substituents selected from halogen atoms and hydroxy groups, C₁₋₄ alkoxy groups which may be optionally substituted by 1, 2 or 3 substituents selected from halogen atoms and hydroxy groups, and C₃-8 cycloalkyl groups which may be optionally substituted by 1, 2 or 3 substituents selected from halogen atoms and hydroxy groups;
• R^{a}, R^{b} and R^{c} independently represent groups selected from hydrogen atoms, halogen atoms, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups;
• R^{d} represents a group selected from hydrogen atoms, halogen atoms, hydroxyl groups, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups, and C₃₋₈ cycloalkoxy groups which may be optionally substituted by 1, 2 or 3 substituents selected from halogen atoms and hydroxy groups;
• one of the groups G³ and G⁴ is a nitrogen atom and the other is a CH group;
• M is a hydrogen atom or a pharmaceutically acceptable cation;
with the proviso that, when at least one of the groups R^{a} and R^{b} represent a hydrogen atom and G² is a group CR^{d}, then R^{d} represents a group selected from C₁₋₄ alkoxy groups which may be optionally substituted by 1, 2 or 3 substituents selected from halogen atoms and hydroxy groups, C₃₋₈ cycloalkoxy groups which may be optionally substituted by 1, 2 or 3 substituents selected from halogen atoms and hydroxy groups;
or a pharmaceutically acceptable salt, or solvate, or N-oxide, or stereoisomer or deuterated derivate thereof;
or azabiphenylaminobenzoic acid derivatives of formula (I-b) 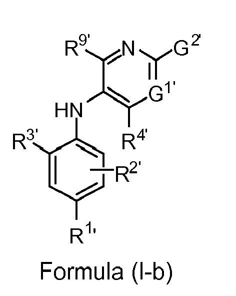 wherein:
• R¹' is selected from the group consisting of hydrogen atoms, halogen atoms, C₁₋₄ alkyl, C₃₋₄ cycloalkyl, -CF₃ and -OCF₃;
• R^{2'} is selected from the group consisting of hydrogen atoms, halogen atoms and C₁₋₄ alkyl group;
• R^{3'} is selected from the group consisting of -COOR⁵, -CONHR⁵, tetrazolyl, -SO₂NHR⁵' and -CONHSO₂R⁵' groups, wherein R^{5'} is selected from the group consisting of a hydrogen atom and linear or branched C₁₋₄ alkyl groups;
• R⁴' is selected from the group consisting of a hydrogen atom, and a C₁₋₄ alkyl group;
• R^{9'} is selected from the group consisting of a hydrogen atom and a phenyl group;
• G^{1'} represents a group selected from N and CR^{6'} wherein R^{6'} is selected from the group consisting of hydrogen atoms, halogen atoms, C₁₋₄ alkyl, C₃₋₄ cycloalkyl, C₁₋₄ alkoxy, -CF₃, -OCF₃, monocyclic N-containing C₅₋₇ heteroaryl, monocyclic N-containing C₃₋₇ heterocyclyl groups and a C₆₋₁₀ aryl group which is optionally substituted with one or more substituents selected from halogen atoms and a C₁₋₄ alkyl group;
• G^{2'} represents a group selected from:
• a hydrogen atom, a hydroxy group, a halogen atom, a C₃₋₄ cycloalkyl group, a C₁₋₄ alkoxy group and -NR^{a'}R^{b'}, wherein R^{a'} represents a C₁₋₄ alkyl group and R^{b'} is selected from a group consisting of C₁₋₄ alkyl group and C₁₋₄ alkoxy-C₁₋₄ alkyl group, or R^{a} and R^{b'} together with the nitrogen atom to which they are attached form a saturated 6 to 8 membered heterocyclic ring optionally containing one oxygen atom as an additional heteroatom;
• a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR^{7'}R^{8'}, wherein R^{7'} and R^{8'} are independently selected from hydrogen atom, linear or branched C₁₋₄ alkyl group, C₃₋₇ cycloalkyl group, or R^{7'} and R^{8'} together with the nitrogen atom to which they are attached form a group of formula 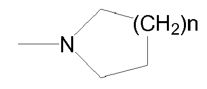 wherein n is an integer from 0 to 3;
and
• a phenyl group which is optionnally substituted by one or more substituents selected from halogen atoms, C₁₋₄ alkyl, hydroxy, C₁₋₄ alkoxy, C₃₋₄ cycloalkyl, C₃₋₄ cycloalkoxy, cyano, -CF₃, -OCF₃, -CONR_{7'}R_{8'}, oxadiazolyl, triazolyl, pyrazolyl and imidazolyl groups, wherein the oxadiazolyl, triazolyl, pyrazolyl and imidazolyl groups are optionally substituted by C₁₋₄ alkyl or C₃₋₇, cycloalkyl group and wherein R_{7'} and R₈, are independently selected from hydrogen atom, linear or branched C₁₋₄ alkyl group, a C₃₋₇ cycloalkyl group, or R_{7'} and R₈, together with the nitrogen atom to which they are attached form a group of formula 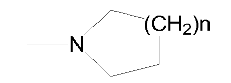 wherein n is an integer from 0 to 3;
• or, when G^{1'} represents CR^{6'}, G^{2'} together with R^{6'} form a non-aromatic
C₅₋₁₀ carbocyclic group or a C₆₋₁₀ aryl group;
or a pharmaceutically acceptable salt, or solvate, or N-oxide, or stereoisomer or deuterated derivate thereof.

3. An amino derivative according to claim 2, wherein in the amino(iso)nicotinic acid derivative of formula (I-a) both groups G¹ represent C(R^{c}) groups, G² represents a C(R^{d}) group, R^{a} is selected from the group consisting of hydrogen atoms, fluorine atoms, chlorine atoms, methyl groups, trifluoromethoxy groups and 2,2,2-trifluoroethoxy groups, R^{b} is selected from the group consisting of hydrogen atoms and fluorine atoms, R¹ is selected from the group consisting of hydrogen, bromine chlorine and fluorine atoms, methyl, ethyl and cyclopropyl groups, R² is selected form the group consisting of hydrogen atoms, fluorine atoms, chlorine atoms and methyl groups, and either G³ represents a group CH and G⁴ represents a nitrogen atom or G³ represents a nitrogen atom and G⁴ represents a group CH.

4. An amino derivative according to claim 3, wherein in the amino(iso)nicotinic acid derivative of formula (I-a) both groups G¹ represent C(R^{c}) groups, G² represents a C(R^{d}) group, R^{a} is selected from the group consisting of fluorine atoms and 2,2,2-trifluoroethoxy groups, R^{b} is selected from the group consisting of hydrogen atoms and fluorine atoms, R¹ is selected from the group consisting of hydrogen, bromine and fluorine atoms, methyl, ethyl and cyclopropyl groups, G³ represents a group CH and G⁴ represents a nitrogen atom.

5. An amino derivative according to claim 2, wherein the amino(iso)nicotinic acid derivative of formula (I-a) is selected from the group consisting of 2-(3'-ethoxy-3-(trifluoromethoxy)biphenyl-4-ylamino)nicotinic acid, 2-(3'-methoxy-3-(trifluoromethoxy)biphenyl-4-ylamino)nicotinic acid, 2-(3'-ethoxy-3,5-difluorobiphenyl-4-ylamino)nicotinic acid, 2-(3,5-difluoro-3'-methoxybiphenyl-4-ylamino)nicotinic acid, lithium 3-(3'-ethoxy-3-fluorobiphenyl-4-ylamino)isonicotinate, lithium 3-(3-fluoro-3'-methoxybiphenyl-4-ylamino)isonicotinate, lithium 3-(3'-methoxy-3-(trifluoromethoxy)biphenyl-4-ylamino)isonicotinate, 2-(3-fluoro-3'-methoxybiphenyl-4-ylamino)-5-methylnicotinic acid, 5-bromo-2-(3,5-difluoro-3'-methoxybiphenyl-4-ylamino)nicotinic acid, 5-cyclopropyl-2-(3-fluoro-3'-methoxybiphenyl-4-ylamino)nicotinic acid, 2-(3,5-difluoro-3'-methoxybiphenyl-4-ylamino)-5-methylnicotinic acid, 2-(3,5-difluoro-3'-methoxybiphenyl-4-ylamino)-5-ethylnicotinic acid, 2-(2'-chloro-3,5-difluorobiphenyl-4-ylamino)nicotinic acid, 2-(3'-cyclopropoxy-3,5-difluorobiphenyl-4-ylamino)nicotinic acid, 2-(3,5-difluoro-2-methylbiphenyl-4-ylamino)nicotinic acid, 5-cyclopropyl-2-(2,5-difluoro-3'-methoxybiphenyl-4-ylamino)nicotinic acid, 5-cyclopropyl-2-(3,5-difluoro-3'-(trifluoromethoxy)biphenyl-4-ylamino)nicotinic acid, 2-(2'-chloro-3,5-difluorobiphenyl-4-ylamino)-5-cyclopropylnicotinic acid or 2-(2,3,5,6-tetrafluoro-3'-methoxybiphenyl-4-ylamino)nicotinic acid, and their pharmaceutically acceptable salts, their solvates, their N-oxides, their stereoisomers or their deuterated derivates thereof.

6. An amino derivative according to claim 5, wherein the amino(iso)nicotinic acid derivative of formula (I-a) is selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid, 2-(3,5-difluoro-2-methylbiphenyl-4-ylamino)nicotinic acid, and their pharmaceutically acceptable salts, their solvates, their N-oxides, their stereoisomers or their deuterated derivates thereof.

7. An amino derivative according to claim 2, wherein in the azabiphenylaminobenzoic acid derivative of formula (I-b) R^{1'} is selected from a hydrogen atom, halogen atom, a methyl group , a CF3 group and a cyclopropyl group, R²' represents a hydrogen atom or a methyl group, R^{3'} is a COOR^{5'} group wherein R^{5'} is selected from the group consisting of a hydrogen atom a methyl group, an ethyl group and a tert-butyl group, R⁴' represents a hydrogen atom, a fluorine atom or a methyl group, R^{9'} represents a hydrogen atom or a phenyl group, G^{1'} is selected from nitrogen atoms and CR^{6'} wherein R^{6'} represents a hydrogen atom, a fluorine atom, a chlorine atom, a methyl group, a trifluoromethyl group, a cyclopropyl group and a phenyl group, and G^{2'} represents a group selected from:
• a hydrogen atom, a hydroxy group, a fluorine atom, a cyclopropyl group, a methoxy group and -NR^{a'}R^{b'}, wherein R^{a'} represents a C₁₋₂ alkyl group and R^{b'} is selected from a group consisting of C₁₋₂ alkyl group and C₁₋₂ alkoxy-C₁₋₂ alkyl group, or R^{a} and R^{b'} together with the nitrogen atom to which they are attached form a saturated 6 to 7 membered heterocyclic ring optionally containing one oxygen atom as an additional heteroatom;
• a monocyclic or bicyclic 5 to 10 membered heteroaromatic ring containing one or two nitrogen atoms which is optionally substituted by one or two halogen atoms, and -CONR^{7'}R^{8'}, wherein R^{7'} and R^{8'} are independently selected from a hydrogen atom, a C₁₋₂ alkyl group, and a cyclopropyl group, or R^{7'} and R⁸' together with the nitrogen atom to which they are attached form a group of formula 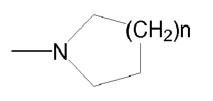 wherein n is 1;
and
• a phenyl group which is optionally substituted by one, two or three substituents selected from fluorine atoms, chlorine atoms, hydroxy, C₁₋₃ alkoxy, cyclopropyl, cyclopropoxy, cyano, -CF₃, -OCF₃, -CONR_{7'}R_{8'} and oxadiazolyl groups, wherein the oxadiazolyl groups are optionally substituted by a methyl group and wherein R_{7'} and R₈, are as defined above;
• or, when G^{1'} represents CR^{6'}, G^{2'} together with R^{6'} form a non-aromatic C₆ carbocyclic group or a phenyl group.

8. An amino derivative according to claim 7, wherein in the azabiphenylaminobenzoic acid derivative of formula (I-b) R^{1'} is selected from a methyl or cyclopropyl group, R^{2'} represents a hydrogen atom, R^{3'} is a COOR^{5'} group wherein R^{5'} is selected from the group consisting of a hydrogen atom and linear or branched C₁₋₄ alkyl groups, R^{4'} represents a hydrogen atom, G^{1'} is selected from nitrogen atoms and CH, C(CH₃) and C(CF₃) groups and G^{2'} represents a cyclopropyl group or a phenyl group optionally substituted with one or two substituents selected from chloro, fluoro, methoxy, ethoxy, isopropoxy, trifluoromethyl, trifluoromethoxy and -CONR^{7'}R^{8'}, wherein R^{7'} is hydrogen and R^{8'} is cyclopropyl or R^{7'} and R^{8'} together with the nitrogen atom to which they are attached form a group of formula 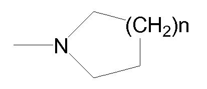 wherein n is 1.

9. An amino derivative according to claim 2, wherein the azabiphenylaminobenzoic acid derivative of formula (I-b) is selected from the group consisting of 5-cyclopropyl-2-(2-phenylpyrimidin-5-ylamino)benzoic acid, 5-(2-carboxy-4-cyclopropylphenylamino)-3-methyl-2-phenylpyridine 1-oxide, 5-methyl-2-(6-(3-(trifluoromethyl)phenyl)pyridin-3-ylamino)benzoic acid, 2-(5-fluoro-6-phenylpyridin-3-ylamino)-5-methylbenzoic acid, 5-cyclopropyl-2-(3'-fluoro-2,4'-bipyridin-5-ylamino)benzoic acid, 2-(5-chloro-6-phenylpyridin-3-ylamino)-5-methylbenzoic acid, 5-cyclopropyl-2-(2-(2-cyclopropylphenyl)pyrimidin-5-ylamino)benzoic acid, 5-cyclopropyl-2-(5-phenylpyridin-3-ylamino) benzoic acid, 2-(5-chloro-2-phenylpyridin-3-ylamino)-5-methylbenzoic acid, 5-cyclopropyl-2-(5,6-diphenylpyridin-3-ylamino)benzoic acid, 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid, 5-methyl-2-((6-(2,3-difluorophenyl)pyridin-3-yl)amino)benzoic acid, 2-(2-(3-cyclopropoxyphenyl)pyrimidin-5-ylamino)-5-cyclopropylbenzoic acid, 5-methyl-2-(6-morpholinopyridin-3-ylamino)benzoic acid, 5-methyl-2-(5-methyl-6-morpholinopyridin-3-ylamino)benzoic acid, 5-cyclopropyl-2-(6-cyclopropyl-5-phenylpyridin-3-ylamino) benzoic acid, 2-(6-(2-cyclopropylphenyl)-5-methylpyridin-3-ylamino)-5-methylbenzoic acid, 2-(6-(2-Cyanophenyl)-5-methylpyridin-3-ylamino)-5-methylbenzoic acid, 2-(2-(3-chlorophenyl)pyrimidin-5-ylamino)-5-cyclopropylbenzoic acid, 5-methyl-2-(6-phenyl-5-(trifluoromethyl)pyridin-3-ylamino)benzoic acid, 5-methyl-2-(5-methyl-6-(piperidin-1-yl)pyridin-3-ylamino)benzoic acid, 2-(6-(azepan-1-yl)-5-methylpyridin-3-ylamino)-5-methylbenzoic acid, 2-(2,3'-bipyridin-5-ylamino)-5-cyclopropylbenzoic acid, 2-(3'-chloro-2,4'-bipyridin-5-ylamino)-5-methylbenzoic acid, 5-methyl-2-(3-methyl-2,2'-bipyridin-5-ylamino)benzoic acid, 2-(6-(3-ethoxyphenyl)-5-methylpyridin-3-ylamino)-5-methylbenzoic acid, 2-(6-(3-methoxyphenyl)-5-methylpyridin-3-ylamino)-5-methylbenzoic acid, 5-methyl-2-(5-methyl-6-phenylpyridin-3-ylamino)benzoic acid, 2-(6-(3-isopropoxyphenyl)-5-methylpyridin-3-ylamino)-5-methylbenzoic acid, 5-cyclopropyl-2-(5-methyl-6-(3-(trifluoromethoxy)phenyl)pyridin-3-ylamino)benzoic acid, 5-cyclopropyl-2-(5-methyl-6-phenylpyridin-3-ylamino)benzoic acid, 2-(6-(2-fluorophenyl)-5-methylpyridin-3-ylamino)-5-methylbenzoic acid, 5-cyclopropyl-2-(6-phenyl-5-(trifluoromethyl)pyridin-3-ylamino)benzoic acid, 5-cyclopropyl-2-(6-(3-methoxyphenyl)-5-(trifluoromethyl)pyridin-3-ylamino)benzoic acid, 2-(6-(2-chlorophenyl)pyridin-3-ylamino)-5-cyclopropylbenzoic acid, 2-(2-(2-chlorophenyl)pyrimidin-5-ylamino)-5-cyclopropylbenzoic acid, 2-(2-(2-chlorophenyl)pyrimidin-5-ylamino)-5-methylbenzoic acid, 2-(6-(2,6-difluorophenyl)pyridin-3-ylamino)-5-methylbenzoic acid, 5-methyl-2-(5-methyl-6-(3-(pyrrolidine-1-carbonyl)phenyl)pyridin-3-ylamino)benzoic acid, 2-(6-(3-(cyclopropylcarbamoyl)phenyl)-5-methylpyridin-3-ylamino)-5-methylbenzoic acid, 5-cyclopropyl-2-(2-(2-fluorophenyl)pyrimidin-5-ylamino)benzoic acid, Methyl 5-cyclopropyl-2-(2-(2-(trifluoromethyl)phenyl)pyrimidin-5-ylamino)benzoate, Methyl 5-cyclopropyl-2-(2-o-tolylpyrimidin-5-ylamino)benzoate, Methyl 2-(2-(2-cyclopropoxyphenyl)pyrimidin-5-ylamino)-5-cyclopropylbenzoate, Methyl 5-cyclopropyl-2-(2-(2,5-difluorophenyl)pyrimidin-5-ylamino)benzoate, Methyl 5-cyclopropyl-2-(2-(2,3-difluorophenyl)pyrimidin-5-ylamino)benzoate, Methyl 2-(2-(5-chloro-2-fluorophenyl)pyrimidin-5-ylamino)-5-cyclopropylbenzoate, tert-Butyl 5-methyl-2-(2-(2-(trifluoromethyl)phenyl)pyrimidin-5-ylamino)benzoate, Methyl 5-cyclopropyl-2-(2-(2-fluoro-5-(trifluoromethyl)phenyl)pyrimidin-5-ylamino)benzoate, Ethyl 2-(3',5'-difluoro-2,4'-bipyridin-5-ylamino)-5-methylbenzoate, tert-Butyl 5-methyl-2-(6-(3-(5-methyl-1,3,4-oxadiazol-2-yl)phenyl)pyridin-3-ylamino)benzoate, tert-Butyl 5-methyl-2-(5-methyl-6-(pyrimidin-5-yl)pyridin-3-ylamino)benzoate, tert-Butyl 2-(6-(2,3-difluorophenyl)pyridin-3-ylamino)-5-methylbenzoate, tert-Butyl 2-(6-(5-fluoro-2-methoxyphenyl)-5-methylpyridin-3-ylamino)-5-methylbenzoate and their pharmaceutically acceptable salts, their solvates, their N-oxides, their stereoisomers or their deuterated derivates thereof.

10. An amino derivative according to claim 9, wherein the azabiphenylaminobenzoic acid derivative of formula (I-b) is selected from the group consisting of 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid, 5-methyl-2-((6-(2,3-difluorophenyl)pyridin-3-yl)amino)benzoic acid, and their pharmaceutically acceptable salts, their solvates, their N-oxides, their stereoisomers or their deuterated derivates thereof.

11. An amino derivative according to any one of claims 1 to 10, which is a DHODH inhibitors having an hDHODH IC₅₀ of about 500 nM or less, as measured using a chromogen reduction assay with DCIP (2,6-dichlorophenol-indophenol).

12. An amino derivative according to any one of claims 1 to 11, wherein the skin disease or disorder is not an autoimmune or inflammatory disease or disorder.

13. An amino derivative according to claim 12, wherein the skin disease or disorder is selected from the group consisting of a) epidermal and follicular tumors (such as basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma); b) sebaceous gland tumors (such as sebaceous carcinoma); c) sweat gland tumors (such as mammary Paget's disease, extramammary Paget's disease, eccrine porocarcinoma, microcystic adnexal carcinoma (MAC) or mucinous carcinoma of the skin); d) nervous system tumors (such as Merkel cell carcinoma (MCC)); e) mesenchymal tumors (such as dermatofibrosarcoma protuberans (DFSP), malignant fibrous histiocytoma (MFH), atypical fibroxanthoma, epithelioid sarcoma, synovial sarcoma, liposarcoma, angiosarcoma, Kaposi's sarcoma, cutaneous lymphoma or multiple myeloma); f) metastatic carcinoma of the skin; g) skin melanoma; h) lamellar ichthyosis; i) acne; or j) hirsutism.

14. An amino derivative according to claim 13, wherein the skin disease or disorder is selected from the group consisting of basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma.

15. An amino derivative according to any one of claims 1 to 11, wherein the amino derivative is topically or transdermally administered.

16. Topical pharmaceutical composition comprising an amino derivative as defined in any one of claims 1 to 11 in association with a pharmaceutically acceptable vehicle or carrier.

17. Topical pharmaceutical composition according to claim 16, wherein the composition is formulated in the form of a cream, a gel, an ointment, a paste, a suspension, a lotion, a foam, a spray, an aerosol or a solution.

18. Topical pharmaceutical composition according to claim 16 or claim 17, wherein the composition further comprises one or more active ingredients selected from the group consisting of (a) antibiotics, (b) antifungal agents, (c) antihistamine agents, (d) antimetabolites, (e) corticosteroids, (f) immunosuppressants, (g) nonsteroidal anti-inflammatory drugs (NSAIDs), (h) vitamin A analogues, and (i) vitamin D analogues.

19. Topical pharmaceutical composition according to any one of claims 16 to 18, wherein the composition further comprises anti-irritants agents, antioxidants agents, buffering agents, chelating agents, emollients, penetration enhancing agents, preservative agents, solubilizing agents, thickening agents, wetting agents or mixtures thereof.

20. A topical pharmaceutical composition according to any one of claims 16 to 19, for use in the treatment of a skin disease or disorder as defined in any of claims 1, and 12 to 14.

21. A method for treating a subject afflicted with a skin disease or disorder as defined in any of claims 1, and 12 to 14, which comprises applying to the affected area of the skin of said subject an effective amount of an amino derivative as defined in any one of claims 1 to 11, or a pharmaceutical composition as defined in any one of claims 16 to 20.

22. Use of an amino derivative as defined in any one of claims 1 to 11 for the manufacture of a medicament for the treatment of a skin disease or disorder as defined in any of claims 1, and 12 to 14.
